# EUROPEAN PATENT APPLICATION

(11) **EP 4 338 755 A1**
(43) Date of publication of application: **20.03.2024**
(21) Application number: 23195548.5
(22) Date of filing: 05.09.2023
(51) Int. Cl.: A61K 48/00, C12N 15/85

(54) **USING MINIVECTORS TO TREAT IDIOPATHIC PULMONARY FIBROSIS**

(30) Priority: 07.09.2022 US 202217930298
(71) Applicant: Twister Biotech, LLC, Houston, TX 77225 (US); Baylor College of Medicine, Houston, TX 77030 (US)
(72) Inventor: ZECHIEDRICH, E. Lynn, Houston, 77030 (US); ARAVELO-SOLIZ, Lirio Milenka, Houston, 77030 (US); FOGG, Jonathan Marcus, Houston, 77030 (US); CATAENESE, Jr, Daniel James, Houston, 77225 (US); COKER, Christopher E., Houston, 77225 (US)
(74) Representative: Barker Brettell LLP

(57) **Abstract**

MiniVectors and compositions containing MiniVectors that target genes implicated in IPF selected from CDH11, STATS, STAT6, FoxM1, MDM2, TGFβ, SMAD, PDGFA, or TLR4 and/or increase intracellular levels of reduced glutathione, relaxin, and p53, are provided, along with uses in the treatment of idiopathic pulmonary fibrosis.

## Description

### FEDERALLY SPONSORED RESEARCH AND DEVELOPMENT

This invention was made with government support under grants R01GM115501, R56AI054830, R01AI054830, and R01CA060651 awarded by the National Institutes of Health. The government has certain rights in the invention.

### JOINT RESEARCH AGREEMENT

Not applicable.

### REFERENCE TO ELECTRONIC SEQUENCE LISTING

This application contains a Sequence Listing which has been submitted electronically in .XML format and is hereby incorporated by reference in its entirety. Said .XML copy, created on October 7, 2022, is named "TWISTER-IPF-003US03.xml" and is 127,146 bytes in size. The sequence listing contained in this .XML file is part of the specification and is hereby incorporated by reference herein in its entirety.

### PRIOR ART

The disclosure generally relates to methods of treating idiopathic pulmonary fibrosis using gene therapy and a combination of multiple methods to deliver gene therapy. It also relates to methods of making DNA MiniVectors, compositions comprising MiniVectors and their treatment uses.

### BACKGROUND OF THE INVENTION

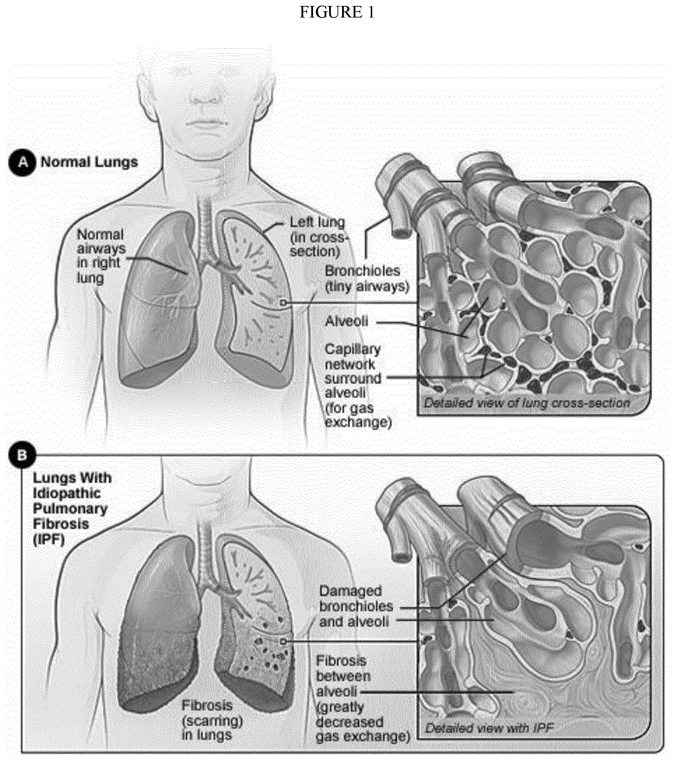

As many as 50,000 new cases of idiopathic pulmonary fibrosis ("IPF") are diagnosed each year in the U.S. and up to 40,000 patients die. Overall, IPF has been estimated to affect 1 out of 200 adults over 65 in the U.S, but young and middle-aged people can also have IPF. The disease is characterized by extensive scarring of the lungs, usually including extensive deposition of collagen and extracellular matrix, usually without an obvious cause. See **FIG. 1****.** Loss of lung architecture leads to shortness of breath and, eventually, respiratory failure. The disease has a mortality rate of 50% within 3-5 years after diagnosis.

In people of any age, IPF can be triggered or worsened by environmental or occupational exposures, including tobacco smoke, infection, and drugs, or can be secondary to autoimmune and inflammatory disorders. Gene variants have been identified that are associated with IPF in more than a third of cases. Additional genes have been found that could potentially be introduced or blocked that would provide improved therapeutics, yet at this time no gene therapy exists for this disease. Current treatments only slow IPF progression and none reverse the disease.

Glutathione is decreased in the damaged tissues of IPF. Glutathione (a tripeptide of glutamic acid, cysteine, and glycine) is an intracellular antioxidant that is used by most organisms to prevent damage caused by reactive oxygen species that are the result of normal cellular metabolism. Reactive oxygen species include free radicals, peroxides, lipid peroxides, and heavy metals. In the process of donating a reducing equivalent to other molecules, glutathione itself becomes oxidized. The reduced form of glutathione can be regenerated from the oxidized form by the action of glutathione reductase. Thus, two ways to increase the amount of intracellular glutathione in these damaged tissues is to either synthesize new glutathione or by increasing the rate of regeneration of reduced glutathione by increasing expression or activity of glutathione reductase. Both of these pathways can be avenues for the treatment of IPF.

One of the most important objectives in gene therapy is the development of highly safe and efficient vector systems to deliver nucleic acids to eukaryotic cells to replace, regulate, or repair genes to prevent or treat disease. Initially, viral-based vector systems were used, most commonly retroviruses or adenoviruses, to deliver the desired genetic payload. Other viruses used as vectors include adeno-associated viruses, lentiviruses, pox viruses, alphaviruses, and herpes viruses. The main advantage of virus-based vectors is that viruses have evolved to physically deliver a genetic payload into cells and this can be readily exploited. The efficiency of delivery into cells is therefore generally higher than non-viral delivery methods, e.g., plasmids.

Viral-based vectors can have disadvantages, however. Viruses can usually infect more than one type of cell and can infect healthy cells as well as diseased cells. Another danger is that the new genetic material might be inserted in the wrong location in the genome, possibly causing cancer or other problems. This has already occurred in clinical trials for X-linked severe combined immunodeficiency (X-SCID) patients. In addition, there is a small chance that viral DNA could unintentionally be introduced into the patient's reproductive cells, thus producing changes that may be passed on to children. Another concern is the lack of regulation and the possibility that transferred genes could be overexpressed, producing so much of the added gene product as to be harmful. Use of viruses is also burdened with concerns of subsequent virus mutation and reactivation. Perhaps most important, viral vectors could cause an immune reaction in the host. Thus, most viral vectors can often only be delivered once because of developed immunity or because subsequent deliveries can produce a stronger immune response and inflammation.

Plasmids could potentially be used instead of viral-based vectors. Plasmids are far less efficient at entering cells than viruses but have utility because they are straightforward to generate and isolate. In fact, clinical trials using intramuscular injection of "naked" DNA plasmid have achieved some success (albeit limited). Unfortunately, expression has been low in comparison to viral vectors-too low to affect disease in many cases.

Numerous studies have shown that the bacterial backbone in plasmids may potentially elicit immune responses as well as cause reduction of transgene expression. CpG motifs occur approximately four times more often in bacterial and viral DNA than in the genomes of eukaryotes. These simple sequence motifs, a cytosine followed by a guanine, are recognized by Toll-like receptor 9 (TLR9), an activator of the innate immune response. Swapping the position of the bases (CpG to GpC) abolishes the immune stimulating activity. Even a single CpG motif on a vector has been reported to be enough to induce an immune response, and more CpG motifs lead to a stronger immune response. Although CpG motifs are underrepresented in eukaryotic genomes, they are still present, but are usually methylated which mitigates their immunogenicity. Plasmids and other bacterial DNA species contain unmethylated CpG motifs and are therefore recognized as foreign DNA when delivered to eukaryotic cells, triggering an immune response.

Plasmids are also subject to transgene silencing in addition to immunotoxicity. Expression of a gene encoded on a plasmid is typically at its highest level shortly after delivery to eukaryotic cells, but this expression subsequently drops to low or even undetectable levels shortly thereafter even though the plasmid remains in the cell. Removal of the bacterial backbone provides more persistent long-term expression, however, there is still no scientific consensus on what elements of the bacterial backbone are responsible for the silencing.

Plasmids require bacterial sequences such as an origin of replication and antibiotic resistance genes to ensure that they are propagated in bacteria during their manufacture. Another potential source of toxicity is the expression of undesired and aberrant protein products because of the bacterial sequences. For example, a commonly used plasmid origin of replication has been reported to contain a cryptic promoter allowing transcription to occur at sites other than the canonical promoter. This results in aberrant, undesired transcripts when the plasmid is delivered to eukaryotic cells. The expression of undesired and unexpected cryptic protein products may induce a significant immune response and has been speculated as an explanation for unforeseen adverse effects in gene therapy trials. Furthermore, the introduction of antibiotic resistance genes, often encoded on plasmids for propagation, is not allowed by some government regulatory agencies, and strongly advised against by others.

Because of these various difficulties, non-viral alternatives to plasmid vectors, such as minicircles and MiniVectors, were developed. These non-viral DNA vectors are small (≤5 kilobase pairs (kb) circular plasmid derivatives that are almost completely devoid of bacterial sequences (such as the antibiotic resistance genes and origins of replication). They have been used as transgene carriers for the genetic modification of mammalian cells, with the advantage that, since they contain no bacterial DNA sequences, they are less likely to suffer from the well documented silencing of transgene expression that often occurs when the transgene is carried on a plasmid containing long bacterial sequences and less likely to elicit an immune response. Therefore, minicircle and MiniVector DNA are less likely to induce an immune reaction or to be silenced, are maintained episomally instead of integrating into the genome, and do not introduce antibiotic resistance genes.

Minicircles and MiniVectors are typically not replicated and become diluted as the host cells divide. For most therapeutic applications, including treatment of IPF, this transience is a desirable feature because once the treatment is finished the vector will ultimately be cleared from the body, reducing the likelihood of long-term side effects. Nevertheless, some therapeutic approaches, for example if the target cells are rapidly dividing, may require increased persistence. This may be achieved by inclusion of a S/MAR sequence (Nehlsen 2006). Minicircles or MiniVectors containing a S/MAR sequence are replicated once each cell cycle, in synchrony with replication of the host cell genome. In addition to facilitating replication, S/MAR sequences also ensure correct segregation of the vectors into the daughter cells, allowing long-term maintenance of the vector.

MiniVectors are similar to minicircles but with some important differences in the final product. Like minicircles, MiniVectors are synthesized from a parent plasmid via site-specific recombination, but they are made and purified differently, resulting in an improved product. US7622252 overcame the problem of yield and purity by transforming the plasmid into a cell suitable for site-specific recombination to occur, under conditions such that topoisomerase IV decatenation activity is inhibited, thereby producing catenated DNA circles, wherein at least one of the circles in each catenane is a supercoiled DNA minicircle of less than about 1 kb in size. We now call vectors made in this way "MiniVectors" to distinguish them from minicircles made using prior methodologies.

Encoding only the genetic payload and short integrase recombination sequences, MiniVectors can be engineered as small as 350, 300, or even 250 base pairs (bp) exclusive of the payload and generated in high yields (the smallest reported minicircle length is 650 bp, but the yield of minicircles this small is unclear). As before, unwanted bacterial sequences are on a discarded miniplasmid. The recombination and purification system used to make MiniVectors is highly optimized, allowing for near complete removal of the parent plasmid, miniplasmid and other DNA species containing undesired bacterial DNA sequences. Plasmid contamination is typically 100-fold lower than the maximum allowable amount recommended by health regulatory agencies (1.5% allowed). Thus, the difference between minicircles and MiniVectors is that MiniVectors have much higher purity and substantially less DNA contaminants.

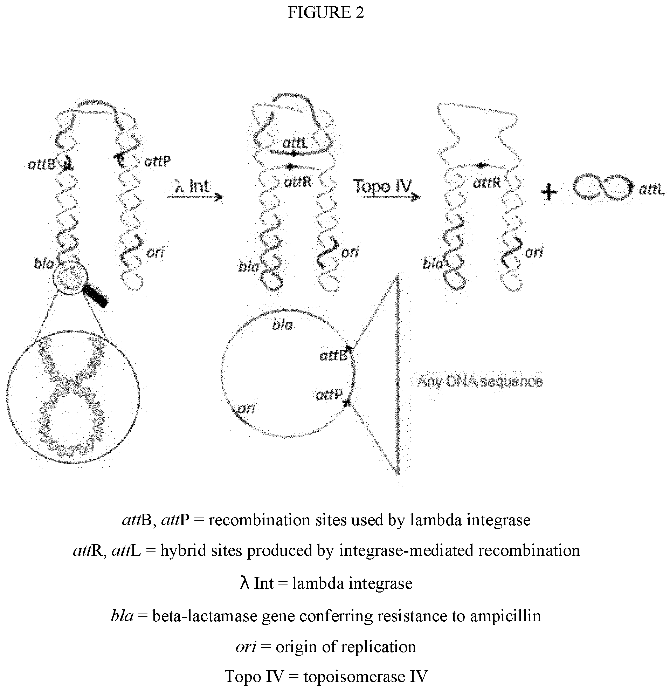

MiniVector preparation usually follows this basic procedure, outlined below and shown in **FIG. 2****:**
1) Production of a "parental" plasmid (bacterial plasmid containing the transgene or other sequence to be delivered, flanked by recombination target sites for *e.g*., -λ-integrase site-specific recombination, *att*B and *att*P-in *Escherichia coli.*
2) Induction of expression of the site-specific recombinase-*e.g*., λ-integrase (Int)-thereby catalyzing strand exchange at the target sites, excising the bacterial sequences, and leaving a minimal vector containing only the desired sequences to be delivered. Other recombinases can be used. Both minicircles and MiniVectors have been produced by λ integrase, φ C31 integrase, Cre recombinase, and FLP recombinase, with specific advantages and disadvantages for each enzyme.
3) Purification of the resulting MiniVectors and removal of contaminants, such as any unrecombined parent plasmid and the excised large circle, the other product of recombination containing the bacterial sequences.

The different DNA species in the MiniVector purification process are typically engineered to be of sufficiently different lengths to be readily separated on the basis of size. The majority of the larger contaminating DNA species may be removed by selective precipitation with polyethylene glycol (PEG). This initial crude separation does not completely remove all DNA contaminants but reducing the fraction of the larger contaminants increases the efficiency of downstream purification steps.

This crude separation is followed by anion exchange chromatography to remove other components, although other steps can be substituted here. This is followed with size-exclusion chromatography (gel-filtration) which separates different-sized species with very high resolution and thus, completely removes any remaining DNA contaminants. In other work, we have modified the size exclusion step to have sequential columns with differing size range separations and have demonstrated that DNA contaminants are less than 0.02% using a gel stain of at least 0.1 ng sensitivity. This provides a unique advantage for the MiniVector system and enables the recovery of an ultrapure preparation of MiniVector of at least 99.98% purity, and probably even higher.

By contrast, a "minicircle" cannot be made as small as a MiniVector and is much less pure, carrying along up to 10% of plasmid and other circle contaminants in the final product. Minicircle purification relies on an endonuclease step leading to degradation of the plasmid in the bacterial host strain during production. This endonuclease step is not 100% efficient and some of the contaminating DNA species remain. Once the minicircle is isolated from the bacteria it is difficult, if not impossible to remove the residual contaminants because the minicircle and the "miniplasmid" containing excised bacterial sequences are of similar size and thus not readily separated.

What is needed in the art are better methods of gene delivery, especially delivery of sequences specific for treating IPF. We are now using the materials and methods described in US7622252 to develop and test a variety of sequences for this use.

### SUMMARY OF THE INVENTION

This application focuses on the treatment of IPF and improving the survival rate of patients by creating MiniVectors to specifically target key pulmonary fibrosis targets and pathways. The MiniVectors expressing inhibitory RNA are designed to target and knock down expression of genes implicated in the development of fibrosis. These include, but are not limited to CDH11, STAT3, STAT6, FoxM1, and MDM2. Additional potential targets are listed in **Table 1** and **Table 6.**

MiniVectors may also be designed to express genes that when expressed inhibit the development and progression of fibrosis. These include but are not limited to genes involved in the pathways of synthesis and maintenance of the reduced form of glutathione (see **Table 8),** genes that encode for the hormone relaxin (see **Table 9),** and the p53 gene (and variants thereof). These may be used instead of MiniVectors expressing inhibitory RNA or in combination therewith.

The MiniVectors can contain a single IPF inhibitory (IPFi) sequence, or it can contain more than one such sequence, although with increasing vector length, some transfection efficiency advantages start to be lost. Thus, in some embodiments, MiniVectors encoding IPFi sequences against multiple targets on the same MiniVector can be used to change the expression of two or more targets simultaneously. In another embodiment, multiple MiniVectors, each encoding IPFi against a different target, can be used in combination to change the expression of two or more targets simultaneously. In other embodiments, MiniVectors can be combined with other gene delivery vectors and/or other therapeutic agents to improve treatment.

While other vectors could be used herein for delivery of IPFi genes or RNA sequences, MiniVectors are preferred, as being easier to manufacture in purity and quantity and safer for use, offering the following advantages over other gene delivery vehicles:
- Minivectors, because they are non-viral, devoid of bacterial sequences, and free of DNA contaminants, induce much less of an immune response than other vectors. The low toxicity allows for repeated delivery of the MiniVector to a patient. This is an attractive and essential feature for an IPF therapy which will require treatment over a prolonged period of time.
- MiniVector transfection efficiency is equal to siRNA and better than plasmid in the several cell types tested. MiniVector DNA transfects every cell type we have tried, including: aortic smooth muscle cells, suspension lymphoma cells and other difficult to transfect cell types.
- MiniVectors encoding shRNA to knockdown gene expression is a better therapeutic approach than delivery of RNA (e.g., synthetic siRNA) because siRNA, shRNA, or miRNA are all rapidly degraded and require constant replenishment. Unlike siRNA, which rapidly degrades, MiniVectors provide a long-lasting effect.
- MiniVectors survive exposure to human serum for more than three times as long as plasmids and survive the shear forces associated with aerosolization. Plasmids are too big to penetrate cells so they require toxic delivery methods. Additionally, they are degraded faster by human serum nucleases and are highly susceptible to the shear forces from nebulization which can linearize and degrade plasmids. Degraded linear DNA can trigger DNA repair and activate apoptosis. The risk of erroneously integrating into the genome of the recipient is also increased if the DNA is linearized.
- MiniVectors, alone with no transfection reagents, withstand nebulization for more than 20 minutes, making them an ideal delivery vector to lungs. Delivery of DNA to the lungs via aerosol is a desirable delivery route, especially for a lung disease such as IPF.
- MiniVectors transfect T-cells, stem cells, and lung cells.

"MiniVector" as used herein, is a double-stranded, supercoiled circular DNA typically lacking a bacterial origin of replication or an antibiotic selection gene, and having a size of about 250 bp up to about 5 kb. It is usually obtained by site-specific recombination of a parent plasmid to eliminate plasmid sequences outside of the recombination sites, but the sizes of the various components are designed to facilitate separation, and the separation is not endonuclease based by definition herein.

Purity levels of MiniVectors are typically much higher than a minicircle preparation and there is usually, by agarose gel electrophoresis analysis with 0.1 ng sensitivity or better stains, no detectible contamination by other DNAs, such as catenanes (linked circles), the other circular recombination product ("mini plasmid"), nor the parent plasmid. Multimeric forms consist of a single circular DNA molecule containing two (or more) copies of the MiniVector sequence, one after the other. These multimeric forms are merely double the desired therapy and may not be considered contaminants by some, but an extra gel filtration step readily separates higher multimers from unit-sized MiniVector.

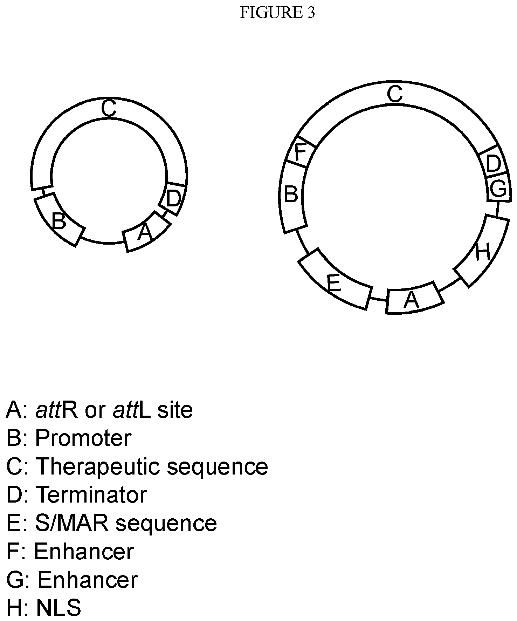

**FIG. 3** schematizes the modular design of MiniVectors. On the left is shown the simplest embodiment of a MiniVector consisting of (A) the hybrid DNA sequences, *att*L or *att*R, that are products of the site-specific recombination, (B) a promoter, (C) the therapeutic sequence to be expressed, and (D) a transcriptional terminator. The MiniVector contains, for example, a nucleic acid molecule with merely the transgene expression cassette (including promoter and a nucleic acid sequence of interest (aka payload), wherein the nucleic acid sequence may be, for example, a gene, or a segment of a gene, a sequence encoding an interfering RNA (*e*.*g*., shRNA, IhRNA, miRNA, IncRNA, piRNA), or a template, for *e*.*g*., homology-directed repair, alteration, or replacement of the targeted DNA sequence).

DNA transfected into eukaryotic cells is occasionally integrated into the genome of the host in approximately one cell per thousand for plasmids. Although this integration frequency is low it still poses a risk of unforeseen and potentially deleterious genetic alterations or even cancer. Therefore, although the integration frequency is low it is important to further mitigate any risk, particularly for a chronic disease such as IPF which will require repeat dosing over an extended period of time. Integration of transfected DNA occurs through homologous recombination and illegitimate integration. The MiniVector itself is designed to contain limited or no homology to the human genome, thereby minimizing integration via homologous recombination mechanisms. It is also typically much shorter in length than plasmids, further reducing the probability of homologous recombination.

Illegitimate integration requires free DNA ends. The probability of chromosomal integration is thus increased if the DNA is linearized. Because of their small size, MiniVectors are much more resistant to the shear forces associated with delivery including nebulization and pneumatic delivery. Delivery of DNA to the lungs should be an efficient method to get the DNA to the diseased cells of the patient. Thus, the ability to withstand the shear forces associated with nebulization is important for IPF. The integration probability of MiniVectors is at least as low as the 5 x 10⁻⁶ rate reported for plasmid integration and likely is much lower, further adding to their benefits in terms of safety. Designed to be delivered locally, any non-target would have to have MiniVector in the non-target cells/tissue to cause an off-target effect. In that way, then, MiniVectors should not have off-target effects. In contrast, many viruses are designed to integrate into the genome, and therefore, there is a major risk of off-target integration.

Optimization of transfection protocols is recommended to gain the full benefits of MiniVectors. We have had good results with a number of transfection reagents including, but not limited to, Lipofectamine^{™}2000 (Life Technologies), Lipofectamine^{™}3000 (Life Technologies), FuGENE^{™} (Promega), Xfect^{™} (Clontech), Effectene^{™} (Qiagen), and GenJet^{™} (SignaGen). We recommend using the standard protocol for the reagent as a starting point, then optimize by varying the amount of DNA transfected. Because of its excellent transfection efficiency and small size (and consequently more copies per unit mass) considerably less MiniVector and reagent should be needed than is typical for larger vectors.

VHH is the variable domain of the heavy chain antibody derived from any member of the biological family *Camelidae,* which includes camels and llamas. VHH is used in this case rather than a human antibody due to its smaller size. VHH is a protein domain about 135-140 amino acids that can be designed to specifically bind any target protein of interest. Like traditional antibody production, VHH can be derived by injecting the target protein into an animal, in this case, an alpaca. The B-cells that produce the antibodies specific to the target protein can be isolated and the sequence of the VHH can be determined using PCR and sequencing techniques. Alternatively, a yeast two-hybrid system using a randomized library of VHH domains can be used to identify which VHH domains bind specifically to the target protein. Other methods could be used as well. Once the amino-acid sequence of the VHH has been determined, a DNA sequence encoding for this VHH can be designed using established methods. Being much smaller than canonical antibodies, the VHH can be readily encoded on a MiniVector, without losing the benefits that arise from the small size of MiniVectors.

Degrons are small protein motifs (about 6-15 amino acids) that are necessary and sufficient for protein degradation. Different sequences of degrons control the rate of protein degradation inside the cell. Degrons come in two varieties, ubiquitin-dependent and ubiquitin-independent, and these protein motifs target a protein for degradation by the proteasome. Ubiquitination is a post-translational modification that is used for multiple purposes, including to "tag" a protein for degradation, change a protein's cellular location, affect a protein's activity, or alter a protein's interactions with other biological molecules. In the case of ubiquitin-dependent degrons, the small protein motif contains a lysine residue that is the site of attachment by ubiquitin. If required, the degron will be encoded on the same MiniVector as the VHH.

Ubiquitin is a 76 amino acid tag that is added to a protein targeted for degradation via a three enzyme cascade that results in the formation of an isopeptide between glycine 76 of ubiquitin and the lysine residue found in the degron. Once formed, additional ubiquitin molecules are attached to the first ubiquitin molecule, and this poly-ubiquitination is what signals the proteasome to actively degrade the target protein. While ubiquitination can occur on a cysteine, serine, threonine, or the N-terminus of a protein, poly-ubiquitination through a lysine in the degron is specific for protein degradation by the proteasome. In the case of ubiquitin-independent degrons, the small protein motif is targeted by mechanisms that are still being investigated, but which ultimately lead to protein degradation by the proteasome.

As used herein, an "IPFi" is an IPF inhibitory sequence, however, the sequence itself does not necessarily inhibit expression of a target gene, as some of the sequences described herein actually encode for and promote expression of beneficial target genes.

As used herein, "shape" encompasses the basic geometric shapes, such as star, rod, disc, and the like, as well as including features such as aspect ratio, local surface roughness, features in all three-dimensions, varied surface curvatures, the potential for creative and diverse biomimicry, numbers of surface appendages, extreme geometries, etc.

As used herein, "a defined geometric shape" means that the MiniVector has a particular geometric shape that is non-transient, *e*.*g*., is retained in solution and *in vivo,* such as *e.g.,* a rod, a star, a hexagon, a cube or rhomboid, or a tetrahedron. It expressly excludes linear or nicked DNAs that freely change shape in solution or ordinary supercoiled DNAs lacking a non-transient shape imposed thereon. Furthermore, the shape is a function of the DNA sequence, and is not externally imposed thereon, *e*.*g*., by histones, capsid proteins, ligands, or micelles, and the like.

As used herein, when we say that > 50% of said MiniVectors have a specific shape, we mean that when visualized by *e*.*g*., scanning electron microscopy, we see that more than half of the vectors have the same shape, although that shape may be viewed from different angles. Shape has been shown to influence cellular localization and uptake of synthetic nanoparticles (*e.g*., gold nanorods). Particles with diameters on the order of microns (interestingly about the same size as a platelet) preferentially displace to the cell free layer (CFL) in the presence of red blood cells (RBCs), while smaller particles do not experience this enhanced localization. Although not yet directly tested with DNA, we anticipate that shape and size will similarly affect the cellular localization and uptake of DNA nanoparticles generated by incorporation of sequence-directed bends into the MiniVector DNA.

As used herein, "non-transient" means that the shape is retained in solution and *in vivo* (unless accidently nicked). Transient shapes, by contrast include the various forms that a simple circular or linear DNA can take in solution, such as random linear DNA shapes, circles that are twisted and distorted in one or more directions. A "non-transient shape" is best assessed by electron microscopy wherein > 50% of the MiniVectors demonstrate the desired shape, preferably >75%, >85%, or better. Alternative methods include atomic force microscopy, scanning electron microscopy, and the like.

As used herein, a "star" shape has a plurality of generally evenly sized and distributed projections, *e*.*g*., six armed stars have been shown to be preferentially delivered to pulmonary tissue. Thus, star shaped MiniVectors may be of particular use herein.

As used herein, the term "gene silencing" refers to a reduction in the expression product of a target gene. Silencing may be complete, in that no final gene product is detectable, or partial, in that a substantial reduction in the amount of gene product occurs.

As used herein, the term "RNA interference," or "RNAi," refers to the process whereby sequence-specific, post-transcriptional gene silencing is initiated by an RNA that is homologous in sequence to the silenced gene. RNAi, which occurs in a wide variety of living organisms and their cells, from plants to humans, has also been referred to as post-transcriptional gene silencing (PTGS) and co-suppression in different biological systems. The sequence-specific degradation of mRNA observed in RNAi, is mediated by small (or short) interfering RNAs (siRNAs).

As known in the art, interfering RNAs can be "small interfering RNAs," or siRNAs, composed of two complementary single-stranded RNAs that form an intermolecular duplex. Interfering RNAs can also be "long hairpin RNAs," or IhRNAs, which are shRNA-like molecules with longer intramolecular duplexes and contain more than one siRNA sequence within the duplex region. As used herein, "shRNA" is short hairpin RNA or small hairpin RNA, and "IhRNA" is long hairpin RNA, both of which can be used to silence target gene expression via RNAi.

As used herein, "miRNA" is microRNA-a small non-coding RNA molecule (containing about 22 nucleotides) found in plants, animals, and some viruses, that functions in RNA silencing and post-transcriptional regulation of gene expression. MicroRNAs are typically encoded within longer primary sequences (pri-miRNA) that are expressed as a long transcript, and that can be more than 1000 nucleotides in length. The long pri-mRNA transcript is subsequently processed by Drosha into pre-miRNA, 60 to 120 nucleotides in length, which folds into a hairpin structure. The pre-miRNA is further cleaved by Dicer to form the mature miRNA. All eukaryotic cells, including human, possess the necessary machinery to manipulate and process these precursor sequences to form the mature miRNA.

As used herein, "IncRNA" are long non-coding RNAs. These IncRNAs are a large and diverse class of transcribed RNA molecules with a length of more than 200 nucleotides that do not encode proteins (or lack >100 amino acid open reading frame). IncRNAs are thought to encompass nearly 30,000 different transcripts in humans, hence IncRNA transcripts account for the major part of the non-coding transcriptome. IncRNA discovery is still at a preliminary stage, but there are many specialized IncRNA databases, which are organized and centralized through RNAcentral (rnacentral.org). IncRNAs can be transcribed as whole or partial natural antisense transcripts (NAT) to coding genes, or located between genes or within introns. Some IncRNAs originate from pseudogenes. IncRNAs may be classified into different subtypes (Antisense, Intergenic, Overlapping, Intronic, Bidirectional, and Processed) according to the position and direction of transcription in relation to other genes.

Piwi-interacting RNA or "piRNA" is the largest class of small non-coding RNA molecules expressed in animal cells. piRNAs form RNA-protein complexes through interactions with P-element Induced Wimpy (PIWI) family proteins. These piRNA complexes have been linked to both epigenetic and post-transcriptional gene silencing of retrotransposons and other genetic elements in germ line cells, particularly those in spermatogenesis. They are distinct from miRNA in size (26-31 nucleotides rather than 21-24 nucleotides), lack of sequence conservation, and increased complexity.

The term "treating" includes both therapeutic treatment and prophylactic treatment (reducing the likelihood of disease development). The term means decrease, suppress, attenuate, diminish, arrest, or stabilize the development or progression of a disease (e.g., a disease or disorder delineated herein), lessen the severity of the disease, or improve the symptoms associated with the disease.

As used herein, the term "effective amount" refers to an amount which, when administered in a proper dosing regimen, is sufficient to treat (therapeutically or prophylactically) the target disorder. For example, an effective amount is sufficient to reduce or ameliorate the severity, duration, or progression of the disorder being treated, prevent the advancement of the disorder being treated, cause the regression of the disorder being treated, or enhance or improve the prophylactic or therapeutic effect(s) of another therapy.

MiniVectors can be delivered in a variety of "pharmaceutically acceptable excipients" including saline, a solvent, a branched or linear polymer (e.g., star polymer, a liposome, a hydrogel, a lipid nanoparticle, a silicon nanoparticle, a mesoporous silica nanoparticle, a naturally occurring vesicle (e.g., an exosome), hybrids of the foregoing, or others. MiniVectors can be conjugated with a variety of ligands, agents, sugars (e.g., GalNac), nucleic acids, peptides, proteins, aptamers, hybrids, or other moieties to improve transfection and/or facilitate specific delivery.

In a particular embodiment, the site-specific recombination sites are selected from the group consisting of: *att*B, *att*P, *lox*P sites, yδ *res* sites, FRT sites *hix*L, *hix*R, TN3 res sites, Tn21 *res* sites, *psi* sites and cer sites, and wherein the site-specific recombination reaction utilizes an enzyme selected from the group consisting of: γε resolvase, Hin recombinase, P1 Cre, yeast 2 micron Flp, Tn3 resolvase, Tn21 resolvase, γ integrase and XerCD.

Site-specific recombination can be performed *in vivo* or *in vitro* using purified components, but *in vivo* methods are preferred, especially using topoisomerase IV inhibition with fluoroquinolones. Topoisomerase IV has both decatenation and supercoil relaxation activities. Inhibition of topoisomerase IV both slows decatenation and increases the supercoiling level in the cell. Integrase-mediated site-specific recombination is strongly supercoiling dependent, therefore increasing supercoiling by inhibiting topoisomerase IV also stimulates recombination. Topoisomerase IV has enough residual decatenation activity, even in the presence of fluoroquinolones, to eventually decatenate the products.

As used herein, "decatenating" can be performed, for example, by treating the catenated product with a restriction enzyme or other sequence-specific endonuclease (*e*.*g*., a homing endonuclease) that only cleaves the larger DNA circle and not the DNA minicircle or MiniVector. However, it is preferred that decatenation occur with residual topoisomerase IV or similar activity, not an endonuclease, as this can be done *in vivo* using the endogenous bacterial topoisomerases and does not require any additional endonuclease recognition sequences or enzyme coding sequences.

The large DNA circle containing the excised bacterial sequences is separated from the supercoiled DNA MiniVectors by any of a number of methods known in the art, e.g., agarose gel electrophoresis, polyacrylamide gel electrophoresis, column chromatography, density centrifugation, and the like, so long as the requisite ultrapurity is achieved.

Although supercoiled MiniVectors are preferred, in one embodiment of the invention, nicked MiniVectors are isolated, or generated using a nicking endonuclease, and subsequently supercoiled by using chemical intercalators (such as ethidium bromide, chloroquine, or netropsin) or proteins (such as HmfB) to induce supercoiling. The nicked strand is repaired using a DNA ligase to trap the supercoiling and the chemical or protein used to introduce supercoiling is subsequently extracted. This process enables MiniVectors with different levels of supercoiling to be generated, from hypernegatively supercoiled to positively supercoiled, and all the possible isomers in between.

By "reducing" the expression of a target protein, we mean a reduction of at least 10%, as the body's own immune response may thereby be sufficient to target and treat the fibrotic cells, particularly in a combination therapy combined with an immune-boosting treatment, such as adding CpG motifs, cytokines (chemokines, interferons, interleukins, lymphokines, and tumor necrosis factors). Preferably, the reduction is at least 20%, 30%, or 40%, but typically a complete knockout is not required, and indeed, can contribute to unwanted side effects, depending on the target protein and its function in the body.

The use of the word "a" or "an" when used in conjunction with the term "comprising" in the claims or the specification means one or more than one, unless the context dictates otherwise. The use of the term "or" in the claims is used to mean "and/or" unless explicitly indicated to refer to alternatives only or if the alternatives are mutually exclusive.

The term "about" means the stated value plus or minus the margin of error of measurement or plus or minus 10% if no method of measurement is indicated.

The terms "comprise," "have," "include," and "contain" (and their variants) are openended linking verbs and allow the addition of other elements when used in a claim. The phrase "consisting of" is closed and excludes all additional elements. The phrase "consisting essentially of" excludes additional material elements but allows the inclusions of non-material elements that do not substantially change the nature of the invention, such as instructions for use, buffers, excipients, and the like. Any claim introduced with "comprising" may be changed to "consisting of" or "consisting essentially of" and vice versa. However, the claims are not repeated with each possible transition phrase in the interest of brevity.

### BRIEF DESCRIPTION OF DRAWINGS

**FIG. 1** Idiopathic Pulmonary Fibrosis.
**FIG. 2** Generation of MiniVector DNA by λ-integrase mediated site-specific recombination. Parent plasmid contains the sequence to be delivered, flanked by *att*B and *att*P, target sites for recombination. The parent plasmid is propagated in the special *E. coli* bacterial host strain, LZ54, harboring λ-integrase (Int) under the tight control of the temperature sensitive cl857 repressor. When the cells have reached a suitable density, expression of Int is switched on by increasing the temperature, leading to denaturation of the cl857 repressor, which prevents Int expression at lower temperatures. Recombination results in a catenated product containing the MiniVector. The products are decatenated, preferably by topoisomerase IV-mediated unlinking, subsequent to the removal of topoisomerase inhibitor following the cell harvest. The deletion product, containing the undesired bacterial sequences, is removed, yielding pure, supercoiled MiniVector product. If desired, the MiniVector can encode *att*R and the deletion product can contain *att*L by switching the positions of *att*B and *att*P in the parent plasmid.
**FIG. 3** Modular design of the MiniVectors. On the left, is shown the minimal therapeutic unit, consisting only of **A)** *att*L or *att*R site (these sites are the products of recombination by integrase), **B)** a promoter, **C)** the therapeutic sequence (*e*.*g*., shRNA encoding sequence), and **D)** transcriptional terminator. Potential sequences for **A, B** and **C** are listed in **Tables 1-3.** The intervening regions can include any other sequence and can range in size from 0 to several thousand base pairs. On the right, is shown a modified version containing additional modules that may be added to provide long-term persistence and expression, improve transfection, and facilitate nuclear localization. Any combination of these additional modules may be added to the essential modules. **E)** S/MAR sequence, if incorporated into the MiniVector, will be placed upstream of the transcriptional unit to utilize the dynamic negative supercoiling generated by transcription. **F, G)** Enhancer sequences may be positioned in a number of locations, depending on the identity of the enhancer. Although the figure shows the enhancers at specific locations, they do not necessarily need to be located immediately upstream or downstream of the therapeutic sequence. They may alternatively be placed some distance from the therapeutic sequence. In nature, enhancer sequences can sometimes be located hundreds of thousands of base pairs away from the transcription start site. Enhancers may also be located on an intron within the transcribed sequence. **H)** Nuclear localization sequences, if incorporated, will be placed downstream of the transcriptional unit. Exemplary payloads are found in **Tables 1** and **6-9** and MiniVector component sequences are provided in Table **2-5.**

### DETAILED DESCRIPTION OF THE INVENTION

The disclosure provides novel MiniVectors used to target and treat pulmonary fibrosis. The invention includes any one or more of the following embodiment(s), in any combination(s) thereof:

| |
|---|
| -A composition comprising a pharmaceutically acceptable excipient and a MiniVector, said MiniVector being a double stranded, supercoiled circular DNA encoding an idiopathic pulmonary fibrosis (IPF) inhibitory sequence (IPFi) that can be expressed in a eukaryotic cell and functions to inhibit or reverse the development of IPF, said MiniVector lacking a bacterial origin of replication and lacking an antibiotic resistance gene and being at least 99%, 99.5%, 99.8%, 99.9% or 99.98% pure of contaminating DNA allowing repeated treatment uses in an IPF patient without causing toxicity or immunogenicity. |
| -A composition comprising a MiniVector in a pharmaceutically acceptable excipient, said MiniVector being a double stranded, supercoiled, nicked, or relaxed circular DNA encoding an IPFi and lacking a bacterial origin of replication and lacking an antibiotic resistance gene, wherein said circular DNA is at least 97% free of parent plasmid DNA or recombination side-products, wherein said IPFi is expressible in human cells and i) inhibits the expression of a human target protein selected from CDH11, STATS, STAT6, FoxM1, MDM2, MDM4, TGFβ, SMAD, PDGFA, or TLR4, or ii) increases the level of a target protein selected from glutathione peroxidase, glutathione reductase, P53 or a P53 variant, or relaxin. |
| -A composition comprising a MiniVector in a pharmaceutically acceptable carrier, said MiniVector being a double stranded, supercoiled circular DNA encoding an IPFi that can be expressed in a eukaryotic cell, wherein said IPFi encodes an inhibitory RNA for a target gene or a VHH-degron for a target protein selected from CDH11, STATS, STAT6, FoxM1, MDM2, MDM4, TGFβ, SMAD, PDGFA, TLR4, or encodes glutathione peroxidase, glutathione reductase, relaxin, P53 or a P53 variant, wherein said MiniVector lacks a bacterial origin of replication and lacks an antibiotic resistance gene and is at least 99% pure of contaminating DNA, wherein said MiniVector is made by: |
| a) engineering a parent plasmid DNA molecule comprising site-specific recombination sites on either side of said IPFi; |
| b) transforming said parent plasmid into a cell suitable for site-specific recombination to occur, under conditions such that topoisomerase IV decatenation activity is inhibited, thereby producing a plurality of catenated DNA circles, wherein at least one of the circles in each catenane is a supercoiled DNA MiniVector of less than about 5 kb in length; |
| c) decatenating the catenated site-specific recombination products with a topoisomerase, thereby releasing the supercoiled DNA MiniVector from the catenanes; and |
| d) isolating the supercoiled DNA MiniVector using PEG precipitation and sequential size exclusion gel-filtration chromatography using resins of differing size range separation. |
| Any composition herein described, wherein said MiniVector is separated from a parent plasmid and recombination side-products on the basis of size, and does not use a sequence-specific endonuclease cleavage in vivo. |
| Any composition herein described, wherein said IPFi encodes one or more of the following: |
| a) an inhibitory RNA for a target gene selected from CDH11, STATS, STAT6, FoxM1, MDM2, MDM4, TGFβ, SMAD, PDGFA, TLR4, or a target from Table 6, alone or in any combination, and wherein expression of said target gene is reduced at least 10% by said inhibitory RNA when said MiniVector is introduced into eukaryotic cells; |
| b) a tissue regenerating gene selected from GCLM and GR, and said gene is expressed when said MiniVector is introduced into eukaryotic cells |
| c) a gene encoding the hormone relaxin, and said gene is expressed when the MiniVector is introduced into eukaryotic cells, and thus the expressed relaxin will display anti-fibrotic effects; |
| d) a gene encoding p53, or variants thereof; |
| e) a VHH-degron for a target protein selected from CDH11, STATS, STAT6, FoxM1, MDM2, MDM4, TGFβ, SMAD, PDGFA, TLR4, alone or in any combination, and wherein levels of said target protein is reduced by at least 10% by proteasome-mediated degradation when said MiniVector is introduced into eukaryotic cells. |
| Any composition herein described, comprising a promoter operably connected to said IPFi operably connected to a terminator. |
| Any composition herein described, comprising a promoter connected to said IPFi operably connected to a terminator, and additionally comprising an enhancer sequence and/or a nuclear localization signal. |
| Any composition herein described, wherein said MiniVector is expressible in a human cell and said IPFi is for a human gene. |
| Any composition herein described, wherein said MiniVector is combined with additional MiniVectors encoding the same single or multiple or additional single or multiple IPFi, or wherein said MiniVector encodes multiple IPFi against the same gene. |
| Any composition herein described, that is made by: |
| a) engineering a parent plasmid DNA molecule comprising site-specific recombination sites on either side of said IPFi; |
| b) transforming said parent plasmid into a cell suitable for site-specific recombination to occur, under conditions such that topoisomerase IV decatenation activity is inhibited, thereby producing a plurality of catenated DNA circles, wherein at least one of the circles in each catenane is a supercoiled DNA MiniVector of less than about 5 kb in length; |
| c) decatenating the catenated site-specific recombination products, thereby releasing the supercoiled DNA MiniVector from the catenanes; and |
| d) isolating the supercoiled DNA MiniVector using a method comprising PEG precipitation of large DNA and two or more sequential size exclusion chromatography gel-filtration resins with differing size range of separations. |
| Any composition herein described, wherein said MiniVectors are 250 bp to 5,000 bp in total length, or are < 250 bp in length, excluding said IPFi. |
| Any composition herein described, wherein said IPFi sequence is codon optimized for humans and/or encodes a human target protein, and/or said MiniVector is CpG-free or CpG minimized as compared with any parent sequence, and/or supercoiled, and /or has a specific DNA sequence-defined shape. |
| A method of treating IPF, comprising delivering any composition herein described to a patient having IPF in an amount effective to treat said IPF. |

MiniVectors encoding shRNA against one or more of the idiopathic pulmonary fibrosis targets will be tested *in vitro* and shown to downregulate the corresponding mRNA and encoded protein in fibroblast cells. As noted, the interfering RNA could be any type of RNAi, but screening of targets may be facilitated by using commercially available Dharmacon^{™} shRNA sequences from Horizon Discovery. Some of our preferred targets are discussed below.

CDH11 or CADHERIN 11 (P55287), also known as OSTEOBLAST CADHERIN or OB-CADHERIN or DHOB or CAD11. The gene is known as CDH11, whereas the protein is CAD11. Cadherins, such as CDH11, are cell surface glycoproteins that mediate Ca²⁺-dependent cell-cell adhesion. These proteins have a molecular mass of about 120 kD and are composed of an extracellular domain at the N-terminal end and a relatively small cytoplasmic domain at the C-terminal end; the two domains are connected by a single membrane-spanning sequence. The extracellular domain consists of five subdomains, each of which contains a cadherin-specific motif. Cadherin expression is regulated spatially as well as temporally. Cadherins are thought to play an important role in development and maintenance of tissues through selective cell-cell adhesion activity and may be involved also in the invasion and metastasis of malignant tumors.

In yet other embodiments, the aqueous capture ammonia includes cations, e.g., as described above. The cations may be provided in the aqueous capture ammonia using any convenient protocol. In some instances, the cations present in the aqueous capture ammonia are derived from a geomass used in regeneration of the aqueous capture ammonia from an aqueous ammonium salt. In addition, and/or alternatively, the cations may be provided by combining an aqueous capture ammonia with a cation source, e.g., as described above.

Cadherin-11 is increased in wound healing and fibrotic cells and could thus be important in pulmonary fibrosis. Immunohistochemical studies demonstrated CDH11 expression on fibroblasts, epithelial cells, and alveolar macrophages of patients with pulmonary fibrosis and mice given bleomycin. CDH11-deficient mice, by contrast, had decreased fibrotic endpoints in the bleomycin model of pulmonary fibrosis compared to wild-type mice. Furthermore, anti-CDH11-neutralizing monoclonal antibodies successfully treated established pulmonary fibrosis induced by bleomycin, and TGF-β levels were reduced in bronchoalveolar lavage (BAL) fluid, BAL cells, and primary alveolar macrophages from CDH11-deficient mice. Mechanistic studies demonstrated that TGF-β up-regulated CDH11 expression on A549 cells, and inhibition of CDH11 expression using siRNA reduced TGF-β-induced epithelial-mesenchymal transition. Together, these results identify CDH11 as a therapeutic target for pulmonary fibrosis.

STAT3 or SIGNAL TRANSDUCER AND ACTIVATOR OF TRANSCRIPTION 3 (P40763), also known as ACUTE-PHASE RESPONSE FACTOR or APRF. The STAT3 gene encodes a transcription factor that plays a critical role in mediating cytokine-induced changes in gene expression. Following activation, members of the STAT family translocate to the nucleus and interact with specific DNA elements.

Phosphorylated STAT-3 was elevated in lung biopsies from patients with idiopathic pulmonary fibrosis and bleomycin (BLM)-induced fibrotic murine lungs. C-188-9, a small molecule STAT-3 inhibitor, decreased pulmonary fibrosis in the intraperitoneal BLM model. Also, TGF-β stimulation of lung fibroblasts resulted in SMAD2/SMAD3-dependent phosphorylation of STAT-3. These findings suggest that STAT-3 is also a therapeutic target for pulmonary fibrosis.

STAT6 or SIGNAL TRANSDUCER AND ACTIVATOR OF TRANSCRIPTION 6 (P42226), also known as STAT, INTERLEUKIN 4-INDUCED or IL4-STAT. STAT6 may also be a useful target as it has been demonstrated to regulate many pathologic features of lung inflammatory responses in animal models including airway eosinophilia, epithelial mucus production, smooth muscle changes, Th2 cell differentiation, and IgE production from B cells. Cytokines IL-4 and IL-13 that are upstream of STAT6 are found elevated in human asthma and clinical trials are underway to therapeutically target the IL-4/IL-13/STAT6 pathway. Additionally, recent work suggests that STAT6 may also regulate lung anti-viral responses and contribute to pulmonary fibrosis.

FoxM1 or FORKHEAD BOX M1 is a transcription factor that plays a key role in regulating cell cycle progression. FoxM1 expression is increased in IPF patients and following radiation- or bleomycin-induced fibrosis in mice. Deletion of the FoxM1 gene protects mice from radiation and bleomycin induced fibrosis. Treatment of fibroblasts with siRNA against FoxM1 prevents differentiation. These results suggest that FoxM1 is necessary for the differentiation and proliferation of fibroblasts. FoxM1 is necessary for the progression of IPF and reducing FoxM1 expression may limit the onset of the disease. Thus, FoxM1 is a therapeutic target for IPF.

Two enzymes are responsible for the synthesis of glutathione. Gamma-glutamylcysteine synthetase (also called Glutathione Cysteine Ligase or GCL) is a two-subunit enzyme (a catalytic subunit, GCLC (P48506), and a modifier subunit, GCLM (P48507)) that hydrolyzes an ATP to link the carbonyl group of the glutamate side chain and amine group of cysteine with a gamma peptide linkage to form gamma-glutamylcysteine. Glutathione synthase then uses an ATP to link a glycine through a normal peptide bond to the carboxyl group cysteine of gamma-glutamylcysteine. GCLC is a 73 kDa catalytic subunit of GCL that can catalyze the reaction alone; however, GCLM is a 31 kDa modifier subunit that increases the activity of GCLC. The overall rate-limiting step in the synthesis of glutathione is GCL and it is due to the lower expression of GCLM, thus GCLM is a potential target for treatment of IPF.

As an antioxidant, glutathione (GSH) contains a thiol group (-SH) that reacts with reactive oxygen species to minimize their chemical damage. Glutathione peroxidase (GP (P07203)) catalyzes the conversion of hydrogen peroxide to water using a reducing equivalent from the reduced form of glutathione (GSH), in the process converting GSH to the oxidized form, glutathione disulfide (GSSG). GSSG is reduced back to GSH by the enzyme glutathione reductase (GR (P00390)), which converts NADP⁺ to NADPH in the process. Human GP is a 203 kDa enzyme and Human GR is 52 kDa, although smaller versions exist in other species.

Relaxin is a polypeptide hormone and member of the insulin/relaxin superfamily. In addition to its role in softening the cervix during pregnancy, it has also been reported to have anti-fibrotic roles. Relaxin-null mice develop widespread fibrosis with ageing, suggesting that relaxin plays a role in preventing fibrosis. The anti-fibrotic effects are attributed to downregulation of collagen production and increasing collagen degradation and secretion in affected tissues. Relaxin is encoded by the RLN2 gene in humans. In other mammals (e.g., mice) that lack the RLN2 gene, relaxin is encoded by the RLN1 gene. Delivering the polypeptide hormone, recombinant human relaxin, also known as serorelaxin, has been tested for a number of fibrotic diseases, including IPF, with limited success to date . The short half-life of the hormone (^{~} 10 minutes in serum), is thought to limit its efficacy. Delivering the RLN2 gene, instead of the polypeptide hormone, should overcome this limitation.

p53 or TUMOR PROTEIN 53, in addition to its well-known role in protecting against cancer has also been implicated in inhibiting the onset of fibrosis. Inhibition of p53 using siRNA leads to increased cellular proliferation in fibroblasts. Similarly, a dominant negative mutant of p53 (which binds to and inhibits wildtype p53) was found to increase bleomycin sensitivity in mice, shortening survival time. Conversely, overexpression of p53 in mice inhibits fibrosis. These results suggest that delivering MiniVectors encoding p53 (either wildtype, a truncated version, or other variant therefore) may be a viable therapeutic option for IPF.

MDM2 or MURINE DOUBLE MINUTE GENE 2 (Q00987). The human homologue is sometimes known as HDM2 but typically MDM2 is used for both the mice and human homologues. MDM2 is an important cellular regulator of p53 and targets p53 for degradation. It also binds to p53 and inhibits its activity to activate transcription of other genes. In addition to being a negative regulator of p53, MDM2 also exerts oncogenic and proinflammatory effects via p53-independent pathways. MDM2 also contributes to TGF-β induced fibroblast activation. Knocking down MDM2 expression using siRNA has been shown to inhibit TGF-β induced fibroblast activation. MDM2 levels are often increased in the lungs of IPF patients and MDM2 has been implicated in the disease. These results suggest that inhibiting expression of MDM2 may be a viable therapeutic approach for IPF. Reducing MDM2 should increase p53 expression and activity and thus may be an alternative to delivering p53. Reducing MDM2 expression will also mitigate the other profibrotic activities of the enzyme.

MDM4 (015151), along with MDM2, contributes to TP53 regulation. It inhibits p53/TP53-and TP73/p73-mediated cell cycle arrest and apoptosis by binding its transcriptional activation domain. And inhibits degradation of MDM2. Can reverse MDM2-targeted degradation of TP53 while maintaining suppression of TP53 transactivation and apoptotic functions. MDM4 sequences that will be evaluated include:

MiniVectors can be labeled, e.g., using a chemical moiety, as desired. Methods for internally labelling circular DNA have been described in the prior art. Representative labels include fluorescent dyes, biotin, cholesterol, modified bases, and modified backbones. Representative dyes include: 6-carboxyfluorescein, 5-/6-carboxyrhodamine, 5-/6-carboxytetramethylrhodamine, 6-carboxy-2'-,4-,4'-,5'-,7-,7'-hexachlorofluorescein, 6-carboxy-2'-,4-,7-,7'-tetrachlorofluorescein, 6-carboxy-4'-,5'-dichloro-2'-,7'-dimethoxyfluorescein, 7-amino-4-methylcoumarin-3-acetic acid, Cascade Blue, Marina Blue, Pacific Blue, Cy3, Cy5, Cy3.5, Cy5.5, IRDye700, IRDye800, BODIPY dye, Texas Red, Oregon Green, Rhodamine Red, Rhodamine Green, and the full range of Alexa Fluor dyes.

Additional modifications can also include modified bases (*e*.*g*., 2-aminopurine, deoxyuracil, methylated bases) or modified backbones (*e*.*g*., phosphorothioates, where one of the non-bridging oxygens is substituted by a sulfur; methyl-phosphonate oligonucleotides).

The purified MiniVectors can be transferred into recipient cells or into a differentiated tissue by transfection using, for example, lipofection, electroporation, cationic liposomes, or any other method of transfection, or any method used to introduce DNA into cells or tissues, for instance, jet injection, sonoporation, electroporation, mechanical acceleration (gene gun, *etc.),* or any other method of transfer.

MiniVector may be delivered in a gel, a matrix, a solution, a nanoparticle, a cell, or other means directly into the lungs or into cells *ex vivo* that are then returned to a patient. Typically, *in vivo* studies use injection or surgical introduction, but any method can be used *ex vivo.* It is well known by those skilled in the art that the term "cell" includes CAR T cells or any such cell therapy.

Delivery solutions can be aqueous solutions, non-aqueous solutions, or suspensions. Emulsions are also possible. Delivery solutions can be magnetic, paramagnetic, magnetically resistant, or non-magnetic. Saline is a preferred delivery solution. The MiniVector therapy could optionally be lyophilized.

Solutions of all types may be combined with other phases such as gasses for purposes of delivery. A typical example would be for the purpose of aerosolization and more specifically control of droplet size and droplet size distribution.

MiniVector delivery can be facilitated by an *ex vivo* or *in vivo* device which meters out delivery quantities locally or systemically, but preferably locally. Said devices can control or influence other desired properties such as temperature, pH, shear, and dispersion uniformity. Such devices will likely have microelectromechanical (MEM) or nanoelectromechanical (NEM) components, and can have multiple purposes ("Combination Devices") *ex vivo* or *in vivo.* Functions afforded by a Combination Device could include therapeutic dispensing and optionally therapeutic atomizing, pH control, heating, cooling, magnetic potential control, sensing of these and other activities, and wireless communication amongst others.

MiniVector therapies could be stored in powder form, gel form, as an emulsion, as a solution, as a precipitate under alcohol, or frozen. To maximize the shelf-life of any MiniVector therapy a variety of preservatives can be employed. Example preservatives include but are not limited to ethyleneglycol-bis(2-aminoethylether)-N,N,N',N'-tetraacetic acid ("EGTA"), ethylenediaminetetraacetic acid ("EDTA"), nuclease inhibitors, protease inhibitors, or any other chelating agent.

To improve the efficacy of the MiniVector-based therapies, they may be administered in combination with other FDA approved therapies. Thus, MiniVectors can be administered before, concurrently, and/or after treatment with small molecule drugs, peptides, antibodies, siRNA, minicircles, ministrings, plasmids, viruses, surgery, or radiation, or any combination and/or timing of administration of three or more of these individual approaches.

Combination therapy could be used in IPF treatment because of the heterogeneity and complexity of the disease. One potential combinatorial therapy will be the concurrent administration of several different MiniVectors, each encoding a different payload described herein. Alternatively, multiple different payloads could be encoded for on the same MiniVector, especially RNAi payloads which are quite small. Each RNAi targets a different pathway that is upregulated and necessary for increased fibrosis. Inhibition of multiple pathways concurrently may be more effective in preventing or reversing fibrosis than if each therapy was administered alone.

Another approach is combining MiniVectors encoding shRNAs against a target(s) with an inhibitor of that target(s), we can thus target two distinct characteristics of pulmonary fibrosis. This combinatory approach may be more effective than reducing gene expression alone and should have lower toxicity and additionally a reduced incidence of resistance. CDH11 inhibitors include Neutralizing Cadherin-11 Humanized Antibody and those described in US20150064168. There are a great many STATS inhibitors, including (1) natural products and derivatives, such as curcumin, resveratrol and others, (2) tyrphostins, (3) platinum-containing complexes, (4) peptidomimetics, and (5) azaspiranes. STAT6 inhibitors include AS 1517499, R-84, and R-76.

Various IPF therapies available for combination with MiniVectors include but are not limited to Pirfenidone (Esbriet^{®}, Pirfenex^{®}, Pirespa^{®}), and nintedanib, etc. Stem cell-derived therapies afford great promise in a variety of medical domains including IPF. Stem cell-derived immunotherapy approaches and others could be combined with MiniVector-derived therapies.

Another combination therapy approach would be to deliver multiple MiniVectors, each encoding a different sequence. Delivering multi-target MiniVectors may produce a synergistic effect and be more effective than a single MiniVector. In addition, delivering multiple MiniVectors against targets on different pathways may reduce toxicity and likelihood of resistance. Both approaches will be tested for feasibility.

Another approach would be to combine MiniVectors encoding VHH-degrons with MiniVectors encoding RNAs against any of the above targets. The RNA and VHH-degron could target the same gene and protein, respectively, or could target different genes and proteins. In both cases, the MiniVectors encoding the RNA and VHH-degron could be administered concurrently or in any order with any other therapy.

Another approach would be to combine MiniVectors encoding any of the above targets with MiniVectors encoding any gene involved in the pathways of synthesis and maintenance of the reduced form of glutathione, namely the catalytic or modifier subunits of Glutathione Cysteine Ligase, Glutathione Reductase, and similar genes. These targets would improve the cellular environment of IPF tissue, where it is known that glutathione levels are lower. MiniVectors encoding the RNA, VHH-degron, or glutathione-related genes could be administered concurrently or in any order with any other therapy.

Another approach would be to combine MiniVectors encoding inhibitory RNAs against either CDH11, STAT3, STAT6, or FoxM1 with MiniVectors encoding p53 to enhance the anti-fibrotic effect of the MiniVector therapy.

Although our proof-of-concept work will proceed with both publicly and commercially available sequences, some of which are wild-type and some of which may target common mutations, we contemplate eventually developing personalized target sequences for each patient. IPF is heterogenous in nature. The DNA sequence and gene expression profiles of an individual patient's IPF can be readily determined through high-throughput DNA sequencing, microarrays, quantitative PCR and RNA sequencing on patient tissue samples. These tests allow to determine which sequences and/or gene product(s) are present or absent, and which genes are abnormally expressed, so that a custom MiniVector can be developed encoding targets specifically tailored to a particular patient. This type of approach can be readily modified as needed and according to treatment outcomes by altering one or more of the sequences encoded on the personalized MiniVector.

MiniVector DNA backbone sequence can be modified to engineer DNA sequence and supercoiling-dependent bends to affect DNA 2-dimensional (if planar) or 3-dimensional shape. This capability is enabled by our understanding of how the torsional strain associated with negative supercoiling of DNA results in localized denaturation in the helical structure of DNA. These localized disruptions modify the properties of DNA, generating a hyperflexible site. This, coupled with the tendency of supercoiled DNA to writhe to relieve torsional strain, results in the formation of a DNA bend at the site at which the helical structure is disrupted. Bending at one site will facilitate bending at other sites through mechanical correlations in the DNA molecule. By careful placement of these bend sites we can potentially design MiniVectors with sequences that strongly favor a particular conformation, such as three-lobed or rod-shaped conformations or more complex geometries (Wang, 2017, Arévalo-Soliz 2020).

Geometries such as, but not limited to, rod-shaped, figure-8 shaped, shapes consisting of one or more looped sections, two, three, four, and five or more-leafed clover-shaped, triangle-shaped, square-shaped, rectangle-shaped, trapezoid-shaped, kite-shaped, both regular and irregular pentagon-shaped, hexagon-shaped, other polygon-shaped, star-shaped, disc-shaped, sphere-shaped, ellipse-shaped, cylinder-shaped, cone-shaped, crescent-shaped, obelisk-shaped, tetrahedron-shaped, hexahedron, octahedron-shaped, dodecahedron-shaped, icosahedron-shaped, pointed shapes, shapes that mimic viral capsids, hybrids of these shapes, convex and concave versions as well of each of these geometries, and the like can be engineered to improve transfection or preferentially target one cell type over another. MiniVector shapes may change or be induced over time or with specific condition (encounter with proteins, salts, cell compartment-specific environment, temperature, pH, *etc.*) from one to another shape.

### PROPHETIC EXAMPLES

Novel therapeutic shRNA sequences (at least five) against each of the primary targets, CDH11, STAT3, STAT6, FoxM1, and MDM2, will be designed using freely available, open access, algorithms (*e*.*g*., siRNA Wizard^{™} Software, siDESIGN Center, *etc.*) and then screened for off-targets using NCBI-BLAST. Alternatively, commercially available sequences (*e*.*g*., Dharmacon^{™} shRNA from Horizon Discovery) can be used for initial proof of concept work.

Knockdown efficiency of the *de novo* shRNAs will be validated using synthetic small interfering RNAs (with the same RNA sequence as the expected shRNA transcripts) that will be transfected into either normal human lung fibroblast cells (NHLF) or diseased IPF human lung fibroblast cells (IPF-HLF) obtained from the American Type Culture Collection (ATCC) using lipofection, polyfection, electroporation, nucleofection, sonoporation, or any other method of nucleic acid delivery for cell culture. Knockdown will be assayed by quantitative real-time PCR (Q-RT-PCR) using SYBR^{™} Green PCR master mix to measure levels of the target mRNA in cell lysates. Knockdown efficiencies of the siRNAs will be compared to validated Dharmacon^{™} shRNA sequences (encoded on pGIPZ plasmid vectors) obtained from Horizon Discovery.

shRNA sequences that demonstrate effective levels of knockdown efficiency (>25% reduction in mRNA levels as determined by quantitative RT-PCR analysis and/or >25% reduction in protein levels as determined by western blot analysis) will be cloned between the *att*B and *att*P recombination sites on the MiniVector generating parent plasmid using standard, well-established molecular cloning techniques. Recombination of parent plasmid to generate MiniVectors is carried out as described in **FIG. 2-3** and in US7622252 or FOGG 2006.

NHLF and IPF-HLF cells will be transfected with MiniVectors encoding the most effective shRNAs against each target. Transfection will be performed using lipofection but can also be achieved by another protocol (polyfection, electroporation, nucleofection, sonoporation, *etc.).* Percent knockdown of mRNA targets across the different shRNA-encoding MiniVectors will be assessed by RT-qPCR using the SYBR^{™} Green PCR master mix. We anticipate knockdown efficiencies to show a >25% reduction in mRNA levels as determined by RT-qPCR analysis and/or >25% reduction in protein levels as determined by western blot analysis. The effects of these knockdown levels will be further measured by assessing the phenotype of normal vs. IPF-diseased lung fibroblasts cells.

The phenotype from the knockdown of CDH11, STATS, STAT6, FoxM1, and MDM2, will be assessed in culture by examining the morphology of NHLF and IPF-HLF cells post-transfection. mRNA transcripts of type I collagen (a marker of IPF whose levels correlate with severity of fibrosis) will be measured by RT-qPCR in lysates of both NHLF and IPF-HLF cells post-transfection. We predict that sustained knock-down of the targets will have the best ability to stop fibrotic growth, possibly cause changes in cell morphology (from fibrotic-looking towards normal) and reduce the collagen production resulting from fibrosis.

Off-target effects and any cytotoxicity resulting from the knockdown of CDH11, STATS, STAT6, FoxM1, or MDM2, will be measured in both NHLF and IPF-HLF cells concurrently in the experiments outlined above. Cell viability and apoptosis will be measured using commercially available kits. mRNA from cell lysates of both NHLF and IPF-HLF cells will be used to do microarray analysis or RNA-Seq to further confirm the lack of off-target effects of the therapies. Any potential shRNA candidate that could display any deleterious level of off-target effects or extreme cytotoxicity will not be pursued *in vivo.*

MiniVectors encoding the best shRNA candidates with demonstrated efficient knockdown and corresponding phenotype in cell culture (cell morphology changes towards normal phenotype, and normal collagen mRNA levels), and that do not display any deleterious off-target effects will be pre-clinically tested *in vivo* in a bleomycin-induced lung fibrosis mouse model and saline-treated control mice.

A combinatorial therapy will consist of the concurrent administration of all, or combinations of, MiniVectors encoding an shRNA against CDH11, STATS, STAT6, FoxM1, or MDM2. Each of these MiniVectors encodes shRNAs that target pathways involved in the development or progression of fibrosis. Thus, concurrent administration has the potential for an enhanced effect of the MiniVector therapy.

A therapy aimed at promoting lung tissue regeneration from fibrosis after shRNA treatment of CDH11, STATS, STAT6, FoxM1, and MDM2, will include the co-administration with MiniVectors encoding genes that could promote lung tissue regeneration (*e*.*g*., Glutathione Cysteine Ligase modifier subunit (GCLM) and Glutathione reductase (GR)). The benefit of this approach will be assessed by looking at cell morphology changes in IPF-HLF towards the phenotype of NHLF cells. Increased cellular glutathione levels can be assessed with standardized enzymatic and fluorometric assays.

Bleomycin mice will be generated by intratracheal administration of bleomycin (3.5 U/kg) to C57Bl/6 mice (mouse strain more susceptible to bleomycin). IPF-free control mice will be given saline. Bleomycin-induced lung fibrosis will be characterized 14 days post-administration by testing lung function, and by Ashcroft scoring analysis of hematoxylin-and-eosin (H&E)-stained mouse lungs (on a few mice).

MiniVectors will be administered to both bleomycin and saline-treated control mice intranasally. Weekly after delivery, lung function will be tested, mice will be sacrificed, and lungs as well as other organs will be harvested for gene expression and histological analysis. RT-qPCR and western blot will be done in lung homogenates for validation of the IPF targets *in vivo.* Rapid amplification of cDNA ends (5'RACE) which detects mRNA cleavage, will be conducted for confirmation of knockdown *in vivo.* Histology of the lungs will be done to assess histopathological changes post-treatment, which include collagen deposition and presence of myofibroblasts (both characteristics of fibrotic disease). Collagen deposition and presence of myofibroblasts will be assessed by tissue staining and immunohistochemistry. Histology of other organs will be done concurrently to assess cytotoxicity or any off-target effects of the therapy. This will allow us to re-formulate the therapy if needed. We predict that the MiniVector shRNA and/or increased glutathione treatment will either stop or reverse lung fibrosis.

Alternatively, MiniVectors encoding a VHH-degron specific to CDH11, STATS, STAT6, FoxM1, or MDM2, may be tested. These MiniVectors would be administered the same as above for the MiniVectors encoding inhibitory RNA against these targets. Since the VHH-degron targets proteins, western blotting would be done on lung homogenates for validation of the IPF targets *in vivo.*

In another example, MiniVectors expressing either human RLN2 or murine RLN1 will be administered to bleomycin and saline-treated control mice intranasally. The human form of relaxin is active in mice; however, rodents can mount an immune response to higher doses of human relaxin over time. Therefore, murine RLN1 may be used as an alternative in proof-of-concept work because it is better tolerated. Weekly after delivery, lung function will be tested, mice will be sacrificed, and lungs as well as other organs will be harvested for gene expression and histological analysis as described above. MiniVectors expressing relaxin may also be tested in combination with MiniVectors encoding one or more shRNAs.

In another example, MiniVectors encoding p53, either the full-length wildtype, a truncated version, or another variant thereof, may be tested. These MiniVectors would be administered the same as above for the MiniVectors encoding inhibitory RNA to both bleomycin and saline-treated control mice intranasally. Weekly after delivery, lung function will be tested, mice will be sacrificed, and lungs as well as other organs will be harvested for gene expression and histological analysis as described above. MiniVectors encoding p53 may also be tested in combination with MiniVectors encoding shRNA.

Dosage, treatment frequency, as well as duration of the therapy will be assessed by the methods described herein and also by measuring knockdown of the target mRNAs and proteins, increased relaxin expression, increased p53 levels, increased glutathione levels, and toxicity.

| **Table 1. Therapeutic sequences to be encoded on MiniVector** | | | | |
|---|---|---|---|---|
| **SEQ ID NO.** | **Gene target** | **Description** | **Dharmacon Cat. No.** | **Mature Antisense** |
| 1 | CDH11 | Cadherin11 | V2LHS_150470 | TACTGTACACTAACTTGGC |
| 2 | | | V2LHS_150474 | TAAATCTTGGTCCATTGGC |
| 3 | | | V3LHS_400950 | AACATTTTCTTACATGTCA |
| 4 | | | V3LHS_400949 | AACACATAAACAATTTCCC |
| 5 | STAT3 | Signal transducer and activator of transcription 3 | V2LHS_88502 | TACCTAAGGCCATGAACTT |
| 6 | | | V3LHS_641819 | ATTGCTGCAGGTCGTTGGT |
| 7 | | | V3LHS_641817 | TGCATGTCTCCTTGACTCT |
| 8 | | | V3LHS_641818 | AAGCTGATAATTCAACTCA |
| 9 | | | V3LHS_645974 | ATCTTTCTG CAG CTTCCGT |
| 10 | | | V3LHS_376018 | TAGTAGTGAACTGGACGCC |
| 11 | | | V3LHS_376016 | ATAGTTGAAATCAAAGTCA |
| 12 | STAT6 | Signal transducer and activator of transcription 6 | V2LHS_153578 | TAGCATATGTCAGAGAGGC |
| 13 | | | V3LHS_369098 | ATCTGTGGAGAGCCATCCT |
| 14 | | | V3LHS_369101 | AG CTCTCCAGTG GTCTCCT |
| 15 | TGFβ1 | Transforming growth factor β1 | V2LHS_111877 | TAGTTGGTGTCCAGGGCTC |
| 16 | | | V3LHS_356823 | TGATGTCCACTTGCAGTGT |
| 17 | | | V3LHS_356824 | ATGCTGTGTGTACTCTGCT |
| 18 | | | V3LHS_356827 | TTCTGGTACAGCTCCACGT |
| 19 | | | V3LHS_356825 | ACTCTGCTTGAACTTGTCA |
| 20 | SMAD | Family of signal transducer and transcriptional modulators | V2LHS_216496 | TAGTAGACAATAGAGCACC |
| 21 | | | V2LHS_197114 | AGACAATAGAGCACCAGTG |
| 22 | | | V2LHS_196344 | TAAGATACAGATGAAATAG |
| 23 | | | V3LHS_317711 | TCCTCATAAGCAACCGCCT |
| 24 | | | V3LHS_317709 | AACTGAGCAAATTCTTGGT |
| 25 | | | V3LHS_317710 | TCGCTGTGTCTTGGAACCA |
| 26 | PGFA | Platelet derived growth factor subunit A | V2LHS_169791 | TCTGTTAATACAGGTAAAG |
| 27 | | | V2LHS_169788 | TTTAGGAAATGATGCATAG |
| 28 | | | V2LHS_169787 | TGGGCTTAAATACTACAGC |
| 29 | | | V2LHS_169786 | AGGTTCAGGAATGTAACAC |
| 30 | | | V2LHS_169790 | AAAGAATTTGGGTTCTGTC |
| 31 | | | V3LHS_340444 | AAATGACCGTCCTGGTCTT |
| 32 | | | V3LHS_340445 | TGACACTGCTCGTGTTGCA |
| 33 | TLR4 | Toll like receptor 4 | V2LHS_171350 | TATTAAGGTAGAGAGGTGG |
| 34 | | | V2LHS_221582 | TTTGTTTCAAATTGGAATG |
| 35 | | | V3LHS_374708 | TCATTTCTAAATTCTCCCA |
| 36 | | | V3LHS_374709 | TACTTTGAATCTTGTTGCT |
| 37 | | | V3LHS_374710 | AGACTACTTGGAAAATGCT |
| 38 | | | V3LHS_374707 | TCTTTACTAGCTCATTCCT |
| 39 | MDM4 | | V2LHS_11941 | TATGTACTGACCTAAATAG |
| 40 | MDM4 | | V2LHS_151660 | ATCTGAATACCAATCCTTC |
| 41 | MDM4 | | V3LHS_356802 | TGAACACTGAGCAGAGGTG |
| 42 | MDM4 | | V3LHS_356797 | AACAGTGAACATTTCACCT |

| **Table 2.** MiniVector elements | | | |
|---|---|---|---|
| Module | Element | Description | Use |
| A | λ-*att*L | *att*L from the λ-integrase system | Recombination sites (product of site-specific recombination used to generate MiniVector). Sequences listed in Table 3. |
| | λ-*att*R | *att*R from the λ-integrase system | |
| | λ-*att*B | *att*B from the λ-integrase system | |
| | λ-*att*P | *att*P from the λ-integrase system | |
| | loxP | loxP site for Cre recombinase | |
| | γδ-res | res site for the γδ (Tn1000) resolvase | |
| | FRT | FRT site for Flp recombinase | |
| | hixL | hixL site for Hin recombinase | |
| | hixR | hixR site for Hin recombinase | |
| | Tn3 res | res site for Tn3 resolvase | |
| | Tn21 res | res site for Tn21 resolvase | |
| | cer | cer site for XerCD system | |
| | psi | psi site for XerCD | |
| | AMY1C | Tissue-specific promoter of human amylase alpha 1C (AMY1C) | Initiation of |
| B | CaMKIIα | Ca2+/calmodulin-dependent protein kinase II alpha promoter | transcription. Includes promoters for RNA polymerase II and RNA polymerase III. Full sequences of selected promoters provided in Table 4. |
| | CMV | Promoter from the human cytomegalovirus (CMV) | |
| | Mini CMV | Minimized version of CMV | |
| | CAG | CMV early enhancer/chicken β actin promoter (CAG). Synthetic hybrid promoter made from a) the CMV early enhancer element, b) the promoter, the first exon and the first intron of chicken beta-actin gene, and c) the splice acceptor of the rabbit beta-globin gene | |
| | Cytokerati n 18 and 19 | Cell-specific promoters of the human keratin 18 and 19 genes. | |
| | EF1α | Strong expression promoter from human elongation factor 1 alpha | |
| | β-actin | Promoter from the (human) beta actin gene | |
| | Kallikrein | Tissue-specific promoter of the kallikrein gene. | |
| | NFK-β | Nuclear factor kappa-light-chain-enhancer of activated B cells (NF-Kβ) | |
| | PGK1 | Promoter from human or mouse phosphoglycerate kinase gene (PGK) | |
| | RSV | Long terminal repeat (LTR) of the rous sarcoma virus (RSV) | |
| | SV40 | Mammalian expression promoter from the simian vacuolating virus 40 | |
| | UBC | Promoter of the human ubiquitin C gene (UBC) | |
| | H1 | Promoter from the human polymerase III RNA promoter | |
| | U6 | Promoter from the human U6 small nuclear promoter | |
| C | shRNA | (DNA) sequence encoding short hairpin RNA (shRNA) transcript. Sequences for use in target validation are listed in the Table 1. Potential therapeutic sequences will be designed *de novo* and optimized for knockdown efficiency. | Knockdown of gene expression through RNA interference |
| | miRNA | (DNA) sequence encoding micro-RNA (miRNA) transcript, including miR-29 | |
| | lhRNA | (DNA) sequence encoding long hairpin RNA (lhRNA) transcript | |
| | lncRNA | (DNA) sequence encoding long non-coding RNA (lncRNA) transcript | Knockdown of gene expression (not RNAi) |
| | piRNA | (DNA) sequence encoding piwi-interacting (piRNA) RNA transcript | |
| | gene | (DNA) sequence encoding the open reading frame or segment of open reading frame of a gene | Expression of gene or segment of gene |
| D | | Transcriptional terminator sequence | |
| E | S/MAR | Scaffold/matrix attached region from eukaryotic chromosomes (Sequences listed in Table 5) | Episomal replication |
| | CpG motifs | Unmethylated deoxycytidyl-deoxyguanosine (CpG) dinucleotides: (Sequences listed in Table 5) | Immunostimulatory activity |
| F/G | β-globin intron | Intron of the human β globin gene (130 bp) | Gene expression enhancer |
| | HGH intron | Intron of the human growth hormone gene (262 bp) | |
| H | SV40 early | Simian virus 40 early promoter (351 bp) | Nuclear localization |
| | promoter | | |
| | NF-κβ | Binding site of nuclear factor kappa-light-chain-enhancer of activated B cells (55 bp (5 repeats of GGGGACTTTCC) SEQ ID NO. 86 | |
| | p53 NLS | Binding site of tumor protein 53 (p53): | |
| | | AGACTGGGCATGTCTGGGCA SEQ ID NO. 87 | |
| | p53 NLS | Binding site of tumor protein 53 (p53): | |
| | | GAACATGTCCCAACATGTTG SEQ ID NO. 88 | |
| | Adenoviru s major late promoter | GGGGCTATAAAAGGG SEQ ID NO. 89 | |

| **Table 3.** Complete sequences for element A (recombination sites) | | |
|---|---|---|
| Site | SEQ ID no | Sequence (5'-3') |
| λ-*att*L | 43. | TCCGTTGAAGCCTGCTTTTTTATACTAAGTTGGCATTATAAAAAAGCATTGCTTATCAATTTGT TGCAACGAACAGGTCACTATCAGTCAAAATAAAATCATTATT |
| λ-*att*R | 44. | AGATGCCTCAGCTCTGTTACAGGTCACTAATACCATCTAAGTAGTTGATTCATAGTGACTGCA TATGTTGTGTTTTACAGTATTATGTAGTCTGTTTTTTATGCAAAATCTAATTTAATATATTGATA TTTATATCATTTTACGTTTCTCGTTCAGCTTTTTTATACTAACTTGAGCGAAACG |
| λ-*att*B | 45. | TCCGTTGAAGCCTGLTTTTTTATACTAACTTGAGCGAAACG |
| λ-*att*P | 46. | AGATGCCTCAGCTCTGTTACAGGTCACTAATACCATCTAAGTAGTTGATTCATAGTGACTGCA TATGTTGTGTTTTACAGTATTATGTAGTCTGTTTTTTATGCAAAATCTAATTTAATATATTGATA TTTATATCATTTTACGTTTCTCGTTCAGCTTTTTTATACTAAGTTGGCATTATAAAAAAGCATTG CTTATCAATTTGTTGCAACGAACAGGTCACTATCAGTCAAAATAAAATCATTATT |
| loxP | 47. | ATAACTTCGTATAGCATACATTATACGAAGTTAT |
| γδ-res | 48. | ATTTTGCAACCGTCCGAAATATTATAAATTATCGCACACATAAAAACAGTGCTGTTAATGTGT CTATTAAATCGATTTTTTGTTATAACAGACACTGCTTGTCCGATATTTGATTTAGGATACATTT TTA |
| FRT | 49. | GAAGTTCCTATTCTCTAGAAAGTATAGGAACTTC |
| hixL | 50. | TTCTTGAAAACCAAGGTTTTTGATAA |
| hixR | 51. | TTTTCCTTTTGGAAGGTTTTTGATAA |
| Tn3 res | 52. | CAACCGTTCGAAATATTATAAATTATCAGACATAGTAAAACGGCTTCGTTTGAGTGTCCATTA AATCGTCATTTTGGCATAATAGACACATCGTGTCTGATATTCGATTTAAGGTACATTT |
| Tn21 res | 53. | GCCGCCGTCAGGTTGAGGCATACCCTAACCTGATGTCAGATGCCATGTGTAAATTGCGTCAG GATAGGATTGAATTTTGAATTTATTGACATATCTCGTTGAAGGTCATAGAGTCTTCCCTGACA T |
| cer | 54. | GGTGCGTACAATTAAGGGATTATGGTAAAT |
| psi | 55. | GGTGCGCGCAAGATCCATTATGTTAAAC |

| **Table 4.** Complete sequences for element B (promoters) | | |
|---|---|---|
| Promoter | SEQ ID No. | Sequence (5'-3') |
| CMV | 56. | GACATTGATTATTGACTAGTTATTAATAGTAATCAATTACGGGGTCATTAGTTCATAGCCCAT |
| | | ATATGGAGTTCCGCGTTACATAACTTACGGTAAATGGCCCGCCTGGCTGACCGCCCAACGA CCCCCGCCCATTGACGTCAATAATGACGTATGTTCCCATAGTAACGCCAATAGGGACTTTCC ATTGACGTCAATGGGTGGAGTATTTACGGTAAACTGCCCACTTGGCAGTACATCAAGTGTA TCATATGCCAAGTACGCCCCCTATTGACGTCAATGACGGTAAATGGCCCGCCTGGCATTATG CCCAGTACATGACCTTATGGGACTTTCCTACTTGGCAGTACATCTACGTATTAGTCATCGCTA TTACCATGGTGATGCGGTTTTGGCAGTACATCAATGGGCGTGGATAGCGGTTTGACTCACG GGGATTTCCAAGTCTCCACCCCATTGACGTCAATGGGAGTTTGTTTTGGCACCAAAATCAAC GGGACTTTCCAAAATGTCGTAACAACTCCGCCCCATTGACGCAAATGGGCGGTAGGCGTGT ACGGTGGGAGGTCTATATAAGCAGAGCT |
| mini-CMV | 57. | CCAAAATCAACGGGACTTTCCAAAATGTCGTAACAACTCCGCCCCATTGACGCAAATGGGC GGTAGGCGTGTACGGTGGGAGGTCTATATAAGCAGAGCT |
| RSV | 58. | GGTGCACACCAATGTGGTGAATGGTCAAATGGCGTTTATTGTATCGAGCTAGGCACTTAAA TACAATATCTCTGCAATGCGGAATTCAGTGGTTCGTCCAATCCATGTCAGACCCGTCTGTTG CCTTCCTAATAAGGCACGATCGTACCACCTTACTTCCACCAATCGGCATGCACGGTGCTTTTT CTCTCCTTGTAAGGCATGTTGCTAACTCATCGTTACCATGTTGCAAGACTACAAGAGTATTG CATAAGACTACATT |
| CAG | 59. | GCGTTACATAACTTACGGTAAATGGCCCGCCTGGCTGACCGCCCAACGACCCCCGCCCATTG ACGTCAATAATGACGTATGTTCCCATAGTAACGCCAATAGGGACTTTCCATTGACGTCAATG GGTGGAGTATTTACGGTAAACTGCCCACTTGGCAGTACATCAAGTGTATCATATGCCAAGT ACGCCCCCTATTGACGTCAATGACGGTAAATGGCCCGCCTGGCATTATGCCCAGTACATGAC CTTATGGGACTTTCCTACTTGGCAGTACATCTACGTATTAGTCATCGCTATTACCATGGTCGA GGTGAGCCCCACGTTCTGCTTCACTCTCCCCATCTCCCCCCCCTCCCCACCCCCAATTTTGTAT TTATTTATTTTTTAATTATTTTGTGCAGCGATGGGGGCGGGGGGGGGGGGGGGGCGCGCG CCAGGCGGGGCGGGGCGGGGCGAGGGGCGGGGCGGGGCGAGGCGGAGAGGTGCGGCG GCAGCCAATCAGAGCGGCGCGCTCCGAAAGTTTCCTTTTATGGCGAGGCGGCGGCGGCGG CGGCCCTATAAAAAGCGAAGCGCGCGGCGGGCG |
| EF1a | 60. | GCTCCGGTGCCCGTCAGTGGGCAGAGCGCACATCGCCCACAGTCCCCGAGAAGTTGGGGG GAGGGGTCGGCAATTGAACCGGTGCCTAGAGAAGGTGGCGCGGGGTAAACTGGGAAAGT GATGTCGTGTACTGGCTCCGCCTTTTTCCCGAGGGTGGGGGAGAACCGTATATAAGTGCAG TAGTCGCCGTGAACGTTCTTTTTCGCAACGGGTTTGCCGCCAGAACACAGGTAAGTGCCGT GTGTGGTTCCCGCGGGCCTGGCCTCTTTACGGGTTATGGCCCTTGCGTGCCTTGAATTACTT CCACGCCCCTGGCTGCAGTACGTGATTCTTGATCCCGAGCTTCGGGTTGGAAGTGGGTGGG AGAGTTCGAGGCCTTGCGCTTAAGGAGCCCCTTCGCCTCGTGCTTGAGTTGAGGCCTGGCC TGGGCGCTGGGGCCGCCGCGTGCGAATCTGGTGGCACCTTCGCGCCTGTCTCGCTGCTTTC GATAAGTCTCTAGCCATTTAAAATTTTTGATGACCTGCTGCGACGCTTTTTTTCTGGCAAGAT AGTCTTGTAAATGCGGGCCAAGATCTGCACACTGGTATTTCGGTTTTTGGGGCCGCGGGCG GCGACGGGGCCCGTGCGTCCCAGCGCACATGTTCGGCGAGGCGGGGCCTGCGAGCGCGG CCACCGAGAATCGGACGGGGGTAGTCTCAAGCTGGCCGGCCTGCTCTGGTGCCTGGCCTCG CGCCGCCGTGTATCGCCCCGCCCTGGGCGGCAAGGCTGGCCCGGTCGGCACCAGTTGCGT GAGCGGAAAGATGGCCGCTTCCCGGCCCTGCTGCAGGGAGCTCAAAATGGAGGACGCGGC GCTCGGGAGAGCGGGCGGGTGAGTCACCCACACAAAGGAAAAGGGCCTTTCCGTCCTCAG CCGTCGCTTCATGTGACTCCACGGAGTACCGGGCGCCGTCCAGGCACCTCGATTAGTTCTCG AGCTTTTGGAGTACGTCGTCTTTAGGTTGGGGGGAGGGGTTTTATGCGATGGAGTTTCCCC ACACTGAGTGGGTGGAGACTGAAGTTAGGCCAGCTTGGCACTTGATGTAATTCTCCTTGGA ATTTGCCCTTTTTGAGTTTGGATCTTGGTTCATTCTCAAGCCTCAGACAGTGGTTCAAAGTTT TTTTCTTCCATTTCAGGTGTCGTGA |
| Human β-actin | 61. | GGCCTCCGCGCCGGGTTTTGGCGCCTCCCGCGGGCGCCCCCCTCCTCACGGCGAGCGCTGC CACGTCAGACGAAGGGCGCAGCGAGCGTCCTGATCCTTCCGCCCGGACGCTCAGGACAGC |
| | | GGCCCGCTGCTCATAAGACTCGGCCTTAGAACCCCAGTATCAGCAGAAGGACATTTTAGGA CGGGACTTGGGTGACTCTAGGGCACTGGTTTTCTTTCCAGAGAGCGGAACAGGCGAGGAA AAGTAGTCCCTTCTCGGCGATTCTGCGGAGGGATCTCCGTGGGGCGGTGAACGCCGATGAT TATATAAGGACGCGCCGGGTGTGGCACAGCTAGTTCCGTCGCAGCCGGGATTTGGGTCGC GGTTCTTGTTTGTGGATCGCTGTGATCGTCACTTGGTGAGTAGCGGGCTGCTGGGCTGGCC GGGGCTTTCGTGGCCGCCGGGCCGCTCGGTGGGACGGAAGCGTGTGGAGAGACCGCCAA GGGCTGTAGTCTGGGTCCGCGAGCAAGGTTGCCCTGAACTGGGGGTTGGGGGGAGCGCA GCAAAATGGCGGCTGTTCCCGAGTCTTGAATGGAAGACGCTTGTGAGGCGGGCTGTGAGG TCGTTGAAACAAGGTGGGGGGCATGGTGGGCGGCAAGAACCCAAGGTCTTGAGGCCTTCG CTAATGCGGGAAAGCTCTTATTCGGGTGAGATGGGCTGGGGCACCATCTGGGGACCCTGA CGTGAAGTTTGTCACTGACTGGAGAACTCGGTTTGTCGTCTGTTGCGGGGGCGGCAGTTAT GGCGGTGCCGTTGGGCAGTGCACCCGTACCTTTGGGAGCGCGCGCCCTCGTCGTGTCGTGA CGTCACCCGTTCTGTTGGCTTATAATGCAGGGTGGGGCCACCTGCCGGTAGGTGTGCGGTA GGCTTTTCTCCGTCGCAGGACGCAGGGTTCGGGCCTAGGGTAGGCTCTCCTGAATCGACAG GCGCCGGACCTCTGGTGAGGGGAGGGATAAGTGAGGCGTCAGTTTCTTTGGTCGGTTTTAT GTACCTATCTTCTTAAGTAGCTGAAGCTCCGGTTTTGAACTATGCGCTCGGGGTTGGCGAGT GTGTTTTGTGAAGTTTTTTAGGCACCTTTTGAAATGTAATCATTTGGGTCAATATGTAATTTT CAGTGTTAGACTAGTAAATTGTCCGCTAAATTCTGGCCGTTTTTGGCTTTTTTGTTAGAC |
| NFK-β | 62. | GCTAGCGGGAATTTCCGGGAATTTCCGGGAATTTCCGGGAATTTCCAGATCTGCCGCCCCG ACTGCATCTGCGTGTTCGAATTCGCCAATGACAAGACGCTGGGCGGGGTTTGTGTCATCAT AGAACTAAAGACATGCAAATATATTTCTTCCGGGGACACCGCCAGCAAACGCGAGCAACGG GCCACGGGGATGAAGCAGAAGCTTGGCA |
| Ubiquitin-C | 63. | GTCTAACAAAAAAGCCAAAAACGGCCAGAATTTAGCGGACAATTTACTAGTCTAACACTGA AAATTACATATTGACCCAAATGATTACATTTCAAAAGGTGCCTAAAAAACTTCACAAAACAC ACTCGCCAACCCCGAGCGCATAGTTCAAAACCGGAGCTTCAGCTACTTAAGAAGATAGGTA CATAAAACCGACCAAAGAAACTGACGCCTCACTTATCCCTCCCCTCACCAGAGGTCCGGCGC CTGTCGATTCAGGAGAGCCTACCCTAGGCCCGAACCCTGCGTCCTGCGACGGAGAAAAGCC TACCGCACACCTACCGGCAGGTGGCCCCACCCTGCATTATAAGCCAACAGAACGGGTGACG TCACGACACGACGAGGGCGCGCGCTCCCAAAGGTACGGGTGCACTGCCCAACGGCACCGC CATAACTGCCGCCCCCGCAACAGACGACAAACCGAGTTCTCCAGTCAGTGACAAACTTCAC GTCAGGGTCCCCAGATGGTGCCCCAGCCCATCTCACCCGAATAAGAGCTTTCCCGCATTAGC GAAGGCCTCAAGACCTTGGGTTCTTGCCGCCCACCATGCCCCCCACCTTGTTTCAACGACCT CACAGCCCGCCTCACAAGCGTCTTCCATTCAAGACTCGGGAACAGCCGCCATTTTGCTGCGC TCCCCCCAACCCCCAGTTCAGGGCAACCTTGCTCGCGGACCCAGACTACAGCCCTTGGCGGT CTCTCCACACGCTTCCGTCCCACCGAGCGGCCCGGCGGCCACGAAAGCCCCGGCCAGCCCA GCAGCCCGCTACTCACCAAGTGACGATCACAGCGATCCACAAACAAGAACCGCGACCCAAA TCCCGGCTGCGACGGAACTAGCTGTGCCACACCCGGCGCGTCCTTATATAATCATCGGCGTT CACCGCCCCACGGAGATCCCTCCGCAGAATCGCCGAGAAGGGACTACTTTTCCTCGCCTGTT CCGCTCTCTGGAAAGAAAACCAGTGCCCTAGAGTCACCCAAGTCCCGTCCTAAAATGTCCTT CTGCTGATACTGGGGTTCTAAGGCCGAGTCTTATGAGCAGCGGGCCGCTGTCCTGAGCGTC CGGGCGGAAGGATCAGGACGCTCGCTGCGCCCTTCGTCTGACGTGGCAGCGCTCGCCGTG AGGAGGGGGGCGCCCGCGGGAGGCGCCAAAACCCGGCGCGGAGGC |
| SV40 | 64. | GGTGTGGAAAGTCCCCAGGCTCCCCAGCAGGCAGAAGTATGCAAAGCATGCATCTCAATTA GTCAGCAACCAGGTGTGGAAAGTCCCCAGGCTCCCCAGCAGGCAGAAGTATGCAAAGCAT GCATCTCAATTAGTCAGCAACCATAGTCCCGCCCCTAACTCCGCCCATCCCGCCCCTAACTCC GCCCAGTTCCGCCCATTCTCCGCCCCATGGCTGACTAATTTTTTTTATTTATGCAGAGGCCGA GGCCGCCTCGGCCTCTGAGCTATTCCAGAAGTAGTGAGGAGGCTTTTTTGGAGGCCTAGGC TTTTGCAAA |
| PGK | 65. | CCGGTAGGCGCCAACCGGCTCCGTTCTTTGGTGGCCCCTTCGCGCCACCTTCTACTCCTCCCC TAGTCAGGAAGTTCCCCCCCGCCCCGCAGCTCGCGTCGTGCAGGACGTGACAAATGGAAGT AGCACGTCTCACTAGTCTCGTGCAGATGGACAGCACCGCTGAGCAATGGAAGCGGGTAGG CCTTTGGGGCAGCGGCCAATAGCAGCTTTGCTCCTTCGCTTTCTGGGCTCAGAGGCTGGGA AGGGGTGGGTCCGGGGGCGGGCTCAGGGGCGGGCTCAGGGGCGGGGCGGGCGCCCGAA GGTCCTCCGGAGGCCCGGCATTCTGCACGCTTCAAAAGCGCACGTCTGCCGCGCTGTTCTCC TCTTCCTCATCTCCGGGCCTTTCGACCTGCAGCC |
| H1 | 66. | AATATTTGCATGTCGCTATGTGTTCTGGGAAATCACCATAAACGTGAAATGTCTTTGGATTT GGGAATCTTATAAGTTCTGTATGAGACCACAGATCCC |
| U6 | 67. | GATCCGACGCCGCCATCTCTAGGCCCGCGCCGGCCCCCTCGCACAGACTTGTGGGAGAAGC TCGGCTACTCCCCTGCCCCGGTTAATTTGCATATAATATTTCCTAGTAACTATAGAGGCTTAA TGTGCGATAAAAGACAGATAATCTGTTCTTTTTAATACTAGCTACATTTTACATGATAGGCTT GGATTTCTATAAGAGATACAAATACTAAATTATTATTTTAAAAAACAGCACAAAAGGAAACT CACCCTAACTGTAAAGTAATTGTGTGTTTTGAGACTATAAATATCCCTTGGAGAAAAGCCTT GTT |

| **Table** 5. Complete sequences for elements E, F and G (accessory sequences) | | |
|---|---|---|
| Element | SEQ ID No | Sequence (5'-3') |
| 250 bp S/MAR | 68. | TCTTTAATTTCTAATATATTTAGAATCTTTAATTTCTAATATATTTAGAATCTTTAATTT CTAATATATTTAGAATCTTTAATTTCTAATATATTTAGAATCTTTAATTTCTAATATATT TAGAATCTTTAATTTCTAATATATTTAGAATCTTTAATTTCTAATATATTTAGAATCTTT AATTTCTAATATATTTAGAATCTTTAATTTCTAATATATTTAGAATCTTTAATTTCTAAT ATATTTAGAA |
| 439 bp S/MAR | 69. | TCTTTAATTTCTAATATATTTAGAATCTTTAATTTCTAATATATTTAGAATCTTTAATTT CTAATATATTTAGAATCTTTAATTTCTAATATATTTAGAATCTTTAATTTCTAATATATT TAGAATCTTTAATTTCTAATATATTTAGAATCTTTAATTTCTAATATATTTAGAATCTTT AATTTCTAATATATTTAGAATCTTTAATTTCTAATATATTTAGAATCTTTAATTTCTAAT ATATTTAGAA |
| (45 bp) Type A Cpg motif | 70. | GGTGCATCGATGCAGCATCGAGGCAGGTGCATCGATACAGGGGGG |
| (24 bp) Type B Cpg motif | 71. | TCGTCGTTTTGTCGTTTTGTCGTT |
| (21 bp) Type C CpG motif | 72. | TCGTCGAACGTTCGAGATGAT |
| β-globin intron | 73. | GTTGGTATCAAGGTTACAAGACAGGTTTAAGGAGACCAATAGAAACTGGGCATGTG GAGACAGAGAAGACTCTTGGGTTTCTGATAGGCACTGACTCTCTCTGCCTATTGGTC TATTTTCCCACCCTTAG |
| Human growth hormone intron | 74. | TTCGAACAGGTAAGCGCCCCTAAAATCCCTTTGGGCACAATGTGTCCTGAGGGGAG AGGCAGCGACCTGTAGATGGGACGGGGGCACTAACCCTCAGGTTTGGGGCTTCTG AATGTGAGTATCGCCATGTAAGCCCAGTATTTGGCCAATCTCAGAAAGCTCCTGGTC CCTGGAGGGATGGAGAGAGAAAAACAAACAGCTCCTGGAGCAGGGAGAGTGCTG GCCTCTTGCTCTCCGGCTCCCTCTGTTGCCCTCTGGTTTC |

| **Table 6:** Additional potential targets for treatment of idiopathic pulmonary fibrosis | |
|---|---|
| Cellular functions | Potential gene targets: |
| Cell adhesion and cell to cell contacts | alpha V beta 6 integrin, Coll a1, Coll a2, Col3a1, Col5a1, Col5a2, Col5a3, Col6a4, Col6a5, Col6a6, Col8a1, Col8a2, Col9a1, Col11a1, Col12a1, Col14a1, Col22a1, Col28a1, EC1 domain of cadherin-11, Connective tissue growth factor, Endostatin, Fibrilin-1, Integrin alpha-4, Integrin alpha-5 beta-1, Integrin Target 2 and 3, avB6, Integrin av-6, Loxl2, PAI-1, platelet-derived growth factor receptor alpha and beta, RANTES-PF4, and thrombin inhibitors. |
| Inflammation and immune system function | CCL2, cytokines, Fms-like tyrosine kinase-3, GPR84, CCR2, CCR6, CCR7, IL-13Rα, interferon gamma, IL-4, IL-12, IL-13, IL-33, Lck, Lyn, monocyte chemotactic protein-1 , monocyte chemotactic and activating factor, NOX1, NOX4, Pentraxin-2, sPLA2, TNF-alpha, Tumor Necrosis factor Receptor-1, and p38 stress-activated kinase pathway. |
| Cell proliferation and growth | amphiregulin, 4E-BP1, EGF, FGF21, FGFR1, FGFR2, FGFR3, hepatocyte growth factor, IGF2, IGFBP5, MDM2, MDM4, mTOR, mTORC1, mTORC1 (and other genes in the mTOR pathway), PDGFA, PDGFC, TGF-beta induced EMT, TGF-beta, TGF-beta2, TGF-beta3, vascular endothelial growth factor receptor, and genes in the Wnt pathway. |
| Pulmonary vasculature | 5HT2B receptor antaqonist, angiopoietin-1, and hypoxia inducible factor. |
| Neuronal and myocardial function | A1 adenosine receptor antagonist, AF219, adenosine A2a receptor, adenosine A2b receptor, KATP channel opener, beta-2 receptor agonistic, Rho-associated coiled-coil kinase 1 and 2, and ST2. |
| Lipid metabolism | fatty acid amide hydrolase, lysophospatidic acid receptor, LPA1, and peroxisome proliferator-activated receptors (PPAR-alpha, PPAR-beta/delta, PPAR-gamma). |
| Global cellular metabolism, gene regulation and cell cycle regulation | Akt (protein kinase B), AMPK, CDK4, CDK6, EGFR, components of the GATOR1 complex (Iml1, Nprl2, Nprl3), components of the GATOR2 complex (mio, NUP44A, Sec13, Wdr24, Wdr59), HER2, histone deactylases, lysyl oxidase-like protein 2, HSP47, LOX, LOXL, poly ADP-ribose synthetase, Pl3-kinase, P4HA2, P4HA3, PLOD2, RXFP1, Src kinase, stratifin, Transient Receptor Potential ion channel, TRPV4, and genes in the ubiquitin pathway. |
| Telomeric maintenance | TINF2, TERC, TERT, DKC1 |
| Response to oxidative stress | SESTRIN 1 (SESN1), SESTRIN 2 (SESN2), SESTRIN 3 (SESN3) |
| Additional targets not fitting in any of the above categories | Adipose-derived Mesenchymal stem cells (MSCs), Autotaxin, BCL-2, C5 convertase (C5a, C5b-9), CF transmembrane conductance regulator, EMT Target (epithelial-to-mesenchymal-transition), FXR, Galectin-3 and other galectins, Intracellular protease, Mitogen-activated protein kinase-2, P2X3, PCOLCE2, ULK1 |

| **Table 7:** Sequences of potential degrons | |
|---|---|
| SEQ ID No. | Amino acid sequence |
| 75. | SSSPVSPADDSGSNS |
| 76. | SKENQSENS |
| 77. | SKENIMRSENS |
| 78. | RTALGDIGN |
| 79. | RNMLANAEN |
| 80. | RAALAVLKS |

| **Table 8:** Genes involved in reduced glutathione synthesis and maintenance | |
|---|---|
| SEQ ID No | cDNA Sequence (5' to 3') |
| 81. | ATGGGGCTGCTGTCCCAGGGCTCGCCGCTGAGCTGGGAGGAAACCAAGCGCCATGC CGACCACGTGCGGCGGCACGGGATCCTCCAGTTCCTGCACATCTACCACGCCGTCA AGGACCGGCACAAGGACGTTCTCAAGTGGGGCGATGAGGTGGAATACATGTTGGTAT CTTTTGATCATGAAAATAAAAAAGTCCGGTTGGTCCTGTCTGGGGAGAAAGTTCTTGA AACTCTGCAAGAGAAGGGGGAAAGGACAAACCCAAACCATCCTACCCTTTGGAGACC AGAGTATGGGAGTTACATGATTGAAGGGACACCAGGACAGCCCTACGGAGGAACAAT GTCCGAGTTCAATACAGTTGAGGCCAACATGCGAAAACGCCGGAAGGAGGCTACTTC TATATTAGAAGAAAATCAGGCTCTTTGCACAATAACTTCATTTCCCAGTACCTTAACAA GAAATATCCGACATAGGAGAGGAGAAAAGGTTGTCATCAATGTACCAATATTTAAGGA CAAGAATACACCATCTCCATTTATAGAAACATTTACTGAGGATGATGAAGCTTCAAGG GCTTCTAAGCCGGATCATATTTACATGGATGCCATGGGATTTGGAATGGGCAATTGCT GTCTCCAGGTGACATTCCAAGCCTGCAGTATATCTGAGGCCAGATACCTTTATGATCA GTTGGCTACTATCTGTCCAATTGTTATGGCTTTGAGTGCTGCATCTCCCTTTTACCGAG GCTATGTGTCAGACATTGATTGTCGCTGGGGAGTGATTTCTGCATCTGTAGATGATAG AACTCGGGAGGAGCGAGGACTGGAGCCATTGAAGAACAATAACTATAGGATCAGTAA ATCCCGATATGACTCAATAGACAGCTATTTATCTAAGTGTGGTGAGAAATATAATGACA TCGACTTGACGATAGATAAAGAGATCTACGAACAGCTGTTGCAGGAAGGCATTGATCA TCTCCTGGCCCAGCATGTTGCTCATCTCTTTATTAGAGACCCACTGACACTGTTTGAA GAGAAAATACACCTGGATGATGCTAATGAGTCTGACCATTTTGAGAATATTCAGTCCA CAAATTGGCAGACAATGAGATTTAAGCCCCCTCCTCCAAACTCAGACATTGGATGGAG AGTAGAATTTCGACCCATGGAGGTGCAATTAACAGACTTTGAGAACTCTGCCTATGTG GTGTTTGTGGTACTGCTCACCAGAGTGATCCTTTCCTACAAATTGGATTTTCTCATTCC ACTGTCAAAGGTTGATGAGAACATGAAGGTAGCACAGAAAAGAGATGCTGTCTTGCA GGGAATGTTTTATTTCAGGAAAGATATTTGCAAAGGTGGCAATGCAGTGGTGGATGGT TGTGGCAAGGCCCAGAACAGCACGGAGCTCGCTGCAGAGGAGTACACCCTCATGAG CATAGACACCATCATCAATGGGAAGGAAGGTGTGTTTCCTGGACTGATCCCAATTCTG AACTCTTACCTTGAAAACATGGAAGTGGATGTGGACACCAGATGTAGTATTCTGAACT ACCTAAAGCTAATTAAGAAGAGAGCATCTGGAGAACTAATGACAGTTGCCAGATGGAT GAGGGAGTTTATCGCAAACCATCCTGACTACAAGCAAGACAGTGTCATAACTGATGAA ATGAATTATAGCCTTATTTTGAAGTGTAACCAAATTGCAAATGAATTATGTGAATGCCC AGAGTTACTTGGATCAGCATTTAGGAAAGTAAAATATAGTGGAAGTAAAACTGACTCAT CCAACTAG |
| 82. | ATGGGCACCGACAGCCGCGCGGCCAAGGCGCTCCTGGCGCGGGCCCGCACCCTGC ACCTGCAGACGGGGAACCTGCTGAACTGGGGCCGCCTGCGGAAGAAGTGCCCGTCC ACGCACAGCGAGGAGGAGTTTCCAGATGTCTTGGAATGCACTGTATCTCATGCAGTA GAAAAGATAAATCCTGATGAAAGAGAAGAAATGAAAGTTTCTGCAAAACTGTTCATTGT AGAATCAAACTCTTCATCATCAACTAGAAGTGCAGTTGACATGGCCTGTTCAGTCCTT GGAGTTGCACAGCTGGATTCTGTGATCATTGCTTCACCTCCTATTGAAGATGGAGTTA ATCTTTCCTTGGAGCATTTACAGCCTTACTGGGAGGAATTAGAAAACTTAGTTCAGAG CAAAAAGATTGTTGCCATAGGTACCTCTGATCTAGACAAAACACAGTTGGAACAGCTG TATCAGTGGGCACAGGTAAAACCAAATAGTAACCAAGTTAATCTTGCCTCCTGCTGTG TGATGCCACCAGATTTGACTGCATTTGCTAAACAATTTGACATACAGCTGTTGACTCAC AATGATCCAAAAGAACTGCTTTCTGAAGCAAGTTTCCAAGAAGCTCTTCAGGAAAGCA TTCCTGACATTCAAGCGCACGAGTGGGTGCCGCTGTGGCTACTGCGGTATTCGGTCA TTGTGAAAAGTAGAGGAATTATCAAATCAAAAGGCTACATTTTACAAGCTAAAAGAAGG GGTTCTTAA |
| 83. | ATGGCCCTGCTGCCCCGAGCCCTGAGCGCCGGCGCGGGACCGAGCTGGCGGCGGG CGGCGCGCGCCTTCCGAGGCTTCCTGCTGCTTCTGCCCGAGCCCGCGGCCCTCACG CGCGCCCTCTCCCGTGCCATGGCCTGCAGGCAGGAGCCGCAGCCGCAGGGCCCGC CGCCCGCTGCTGGCGCCGTGGCCTCCTATGACTACCTGGTGATCGGGGGCGGCTCG GGCGGGCTGGCCAGCGCGCGCAGGGCGGCCGAGCTGGGTGCCAGGGCCGCCGTG GTGGAGAGCCACAAGCTGGGTGGCACTTGCGTGAATGTTGGATGTGTACCCAAAAAG GTAATGTGGAACACAGCTGTCCACTCTGAATTCATGCATGATCATGCTGATTATGGCT TTCCAAGTTGTGAGGGTAAATTCAATTGGCGTGTTATTAAGGAAAAGCGGGATGCCTA TGTGAGCCGCCTGAATGCCATCTATCAAAACAATCTCACCAAGTCCCATATAGAAATC ATCCGTGGCCATGCAGCCTTCACGAGTGATCCCAAGCCCACAATAGAGGTCAGTGGG AAAAAGTACACCGCCCCACACATCCTGATCGCCACAGGTGGTATGCCCTCCACCCCT CATGAGAGCCAGATCCCCGGTGCCAGCTTAGGAATAACCAGCGATGGATTTTTTCAG CTGGAAGAATTGCCCGGCCGCAGCGTCATTGTTGGTGCAGGTTACATTGCTGTGGAG ATGGCAGGGATCCTGTCAGCCCTGGGTTCTAAGACATCACTGATGATACGGCATGAT AAGGTACTTAGAAGTTTTGATTCAATGATCAGCACCAACTGCACGGAGGAGCTGGAGA ACGCTGGCGTGGAGGTGCTGAAGTTCTCCCAGGTCAAGGAGGTTAAAAAGACTTTGT CGGGCTTGGAAGTCAGCATGGTTACTGCAGTTCCCGGTAGGCTACCAGTCATGACCA TGATTCCAGATGTTGACTGCCTGCTCTGGGCCATTGGGCGGGTCCCGAATACCAAGG ACCTGAGTTTAAACAAACTGGGGATTCAAACCGATGACAAGGGTCATATCATCGTAGA CGAATTCCAGAATACCAACGTCAAAGGCATCTATGCAGTTGGGGATGTATGTGGAAAA GCTCTTCTTACTCCAGTTGCAATAGCTGCTGGCCGAAAACTTGCCCATCGACTTTTTG AATATAAGGAAGATTCCAAATTAGATTATAACAACATCCCAACTGTGGTCTTCAGCCAC CCCCCTATTGGGACAGTGGGACTCACGGAAGATGAAGCCATTCATAAATATGGAATA GAAAATGTGAAGACCTATTCAACGAGCTTTACCCCGATGTATCACGCAGTTACCAAAA GGAAAACAAAATGTGTGATGAAAATGGTCTGTGCTAACAAGGAAGAAAAGGTGGTTG GGATCCATATGCAGGGACTTGGGTGTGATGAAATGCTGCAGGGTTTTGCTGTTGCAG TGAAGATGGGAGCAACGAAGGCAGACTTTGACAACACAGTCGCCATTCACCCTACCT CTTCAGAAGAGCTGGTCACACTTCGTTGA |

| **Table 9:** Genes encoding relaxin | | | |
|---|---|---|---|
| | | SEQ ID No | cDNA Sequence (5' to 3') |
| | RLN2 (human) | 84. | ATGCCTCGCCTGTTTTTTTTCCACCTGCTAGGAGTCTGTTTACTACTGAA CCAATTTTCCAGAGCAGTCGCGGACTCATGGATGGAGGAAGTTATTAAA TTATGCGGCCGCGAATTAGTTCGCGCGCAGATTGCCATTTGCGGCATG AGCACCTGGAGCAAAAGGTCTCTGAGCCAGGAAGATGCTCCTCAGACA CCTAGACCAGTGGCAGAAATTGTGCCATCCTTCATCAACAAAGATACAG AAACCATAAATATGATGTCAGAATTTGTTGCTAATTTGCCACAGGAGCT GAAGTTAACCCTGTCTGAGATGCAGCCAGCATTACCACAGCTACAACAA CATGTACCTGTATTAAAAGATTCCAGTCTTCTCTTTGAAGAATTTAAGAA ACTTATTCGCAATAGACAAAGTGAAGCCGCAGACAGCAGTCCTTCAGAA TTAAAATACTTAGGCTTGGATACTCATTCTCGAAAAAAGAGACAACTCTA CAGTGCATTGGCTAATAAATGTTGCCATGTTGGTTGTACCAAAAGATCT CTTGCTAGATTTTGCTGA |
| | RLN1 (mouse) | 85. | ATGTCCAGCAGATTTTTGCTCCAGCTCCTGGGGTTCTGGCTATTGCTGA GCCAGCCTTGCAGGACGCGAGTCTCGGAGGAGTGGATGGACGGATTC ATTCGGATGTGCGGCCGTGAATATGCCCGTGAATTGATCAAAATCTGC GGGGCCTCCGTGGGAAGATTGGCTTTGAGCCAGGAGGAGCCAGCTCT GCTTGCCAGGCAAGCCACTGAAGTTGTGCCATCCTTCATCAACAAAGAT GCAGAGCCTTTCGATACGACGCTGAAATGCCTTCCAAATTTGTCTGAAG AGCTCAAGGCAGTACTGTCTGAGGCTCAGGCCTCGCTCCCAGAGCTAC |
| | | | AACACGCACCTGTGTTGAGCGATTCTGTTGTTAGCTTGGAAGGCTTTAA GAAAACTCTCCATGATAAACTGGGTGAAGCAGAAGACGGCAGTCCTCC AGGGCTTAAATACTTGCAATCAGATACCCATTCACGGAAAAAGAGGGAG TCTGGTGGATTGATGAGCCAGCAATGTTGCCACGTCGGTTGTAGCAGA AGATCTATTGCTAAACTCTATTGC |

The following references are incorporated by reference in their entirety for all purposes.

Catanese, D. J., et al., Supercoiled MiniVector DNA resists shear forces associated with gene therapy delivery, Gene Ther. 19(1): 94-100 (2012).

Darquet A.M., et al., Minicircle: an improved DNA molecule for in vitro and in vivo gene transfer, Gene Ther. 6: 209-218 (1999).

Fogg, J.M., et al., Exploring writhe in supercoiled minicircle DNA. J. Phys.-Condens. Matter 18: S145-S159 (2006).

Hardee, C.L., Advances in Non-Viral DNA Vectors for Gene Therapy, Genes 8:65 (2017).

Nehlsen, K., et al., "Replicating minicircles: Generation of nonviral episomes for the efficient modification of diving cells." Gene Therapy and Molecular Biology 10: 233-244 (2006).

Wang, Q., et al., Influence of DNA sequence on the structure of minicircles under torsional stress, Nucleic Acids Research 45: 7633-7642 (2017).

Arévalo-Soliz, L.M., Hardee, C.L., Fogg, J.M., Corman, N.R., Noorbakhsh, C., and Zechiedrich, L. Improving therapeutic potential of non-viral minimized DNA vectors. Cell Gene Ther. Insights 6: 1489-1505 (2020).

Zhao N., et al., Transfection of shRNA-encoding MiniVector DNA of a few hundred base pairs to regulate gene expression in lymphoma cells, Gene Ther. 18(3):220-4 (2011).

US20150376645, US20140056868, 61/653,279, filed May 30, 2012, Supercoiled MiniVectors as a tool for DNA repair, alteration and replacement.

US8460924, US8729044, US9267150, US20110160284, US20120302625, US20130316449, 61/252,455, filed Oct. 16, 2009, Supercoiled MiniVectors^{™} for gene therapy applications.

US7622252, US20070020659, 60/689,298, filed Jun. 10, 2005, Generation of minicircle DNA with physiological supercoiling.

US20060211117 Methods of making minicircles.

WO1994009127 Supercoiled minicircle DNA as a unitary promoter vector.

WO2002083889 Methods for the production of minicircles.

## Claims

1. A composition comprising a pharmaceutically acceptable excipient and a MiniVector, said MiniVector being a double stranded, supercoiled circular DNA encoding an idiopathic pulmonary fibrosis (IPF) inhibitory sequence (IPFi) that can be expressed in a eukaryotic cell and functions to inhibit or reverse the development of IPF, said MiniVector lacking a bacterial origin of replication and lacking an antibiotic resistance gene and being at least 99%, 99.5%, 99.8%, 99.9% or 99.98% pure of contaminating DNA allowing repeated treatment uses in an IPF patient without causing toxicity or immunogenicity.

2. The composition claim 1, wherein said MiniVector is separated from a parent plasmid and recombination side-products on the basis of size, and does not use a sequence-specific endonuclease cleavage *in vivo.*

3. The composition of claim 2, wherein said IPFi encodes one or more of the following:
a) an inhibitory RNA for a target gene selected from CDH11, STATS, STAT6, FoxM1, MDM2, MDM4, TGFβ, SMAD, PDGFA, TLR4, or a target from Table 6, alone or in any combination, and wherein expression of said target gene is reduced at least 10% by said inhibitory RNA when said MiniVector is introduced into eukaryotic cells;
b) a tissue regenerating gene selected from GCLM and GR, and said gene is expressed when said MiniVector is introduced into eukaryotic cells
c) a gene encoding the hormone relaxin, and said gene is expressed when the Minivector is introduced into eukaryotic cells, and thus the expressed relaxin will display anti-fibrotic effects;
d) a gene encoding p53, or variants thereof;
e) a VHH-degron for a target protein selected from CDH11, STATS, STAT6, FoxM1, MDM2, MDM4, TGFβ, SMAD, PDGFA, TLR4, alone or in any combination, and wherein levels of said target protein is reduced by at least 10% by proteasome-mediated degradation when said MiniVector is introduced into eukaryotic cells.

4. The composition of claim 3, comprising a promoter operably connected to said IPFi operably connected to a terminator.

5. The composition of claim 3, comprising a promoter connected to said IPFi operably connected to a terminator, and additionally comprising an enhancer sequence and/or a nuclear localization signal.

6. The composition of claim 2, wherein said MiniVector is expressible in a human cell and said IPFi is for a human gene; or
wherein said MiniVector is combined with additional MiniVectors encoding the same single or multiple or additional single or multiple IPFi; or
wherein said MiniVector encodes multiple IPFi against the same gene.

7. The MiniVector of claim 1, that is made by:
a) engineering a parent plasmid DNA molecule comprising site-specific recombination sites on either side of said IPFi;
b) transforming said parent plasmid into a cell suitable for site-specific recombination to occur, under conditions such that topoisomerase IV decatenation activity is inhibited, thereby producing a plurality of catenated DNA circles, wherein at least one of the circles in each catenane is a supercoiled DNA MiniVector of less than about 5 kb in length;
c) decatenating the catenated site-specific recombination products, thereby releasing the supercoiled DNA MiniVector from the catenanes; and
d) isolating the supercoiled DNA MiniVector using a method comprising PEG precipitation of large DNA and two or more sequential size exclusion chromatography gel-filtration resins with differing size range of separations.

8. The composition of claim 1, wherein said MiniVectors are 250 bp to 5,000 bp in total length; or
wherein said MiniVectors are < 250 bp in length, excluding said IPFi.

9. A composition comprising a MiniVector in a pharmaceutically acceptable excipient, said MiniVector being a double stranded, supercoiled, nicked, or relaxed circular DNA encoding an IPFi and lacking a bacterial origin of replication and lacking an antibiotic resistance gene, wherein said circular DNA is at least 99.98% free of parent plasmid DNA or recombination side-products, wherein said IPFi is expressible in human cells and i) inhibits the expression of a human target protein selected from CDH11, STATS, STAT6, FoxM1, MDM2, MDM4, TGFβ, SMAD, PDGFA, or TLR4, or ii) increases the level of a target protein selected from glutathione peroxidase, glutathione reductase, P53 or a P53 variant, or relaxin.

10. The composition of claim 9, wherein said MiniVectors are 250 bp to 5,000 bp in total length; or
wherein said MiniVectors are < 250 bp in length, excluding said IPFi.

11. The composition of claim 9, wherein said IPFi sequence is codon optimized for humans and/or encodes a human target protein.

12. The composition of claim 9, wherein said MiniVector is CpG-free or CpG minimized as compared with any parent sequence.

13. The composition of claims 9, which is supercoiled; or
which is a specific DNA sequence-defined shape.

14. A composition comprising a MiniVector in a pharmaceutically acceptable carrier, said MiniVector being a double stranded, supercoiled circular DNA encoding an IPFi that can be expressed in a eukaryotic cell, wherein said IPFi encodes an inhibitory RNA for a target gene or a VHH-degron for a target protein selected from CDH11, STAT3, STAT6, FoxM1, MDM2, MDM4, TGFβ, SMAD, PDGFA, TLR4, or encodes glutathione peroxidase, glutathione reductase, relaxin, P53 or a P53 variant, wherein said MiniVector lacks a bacterial origin of replication and lacks an antibiotic resistance gene and is at least 99% pure of contaminating DNA, wherein said MiniVector is made by:
a) engineering a parent plasmid DNA molecule comprising site-specific recombination sites on either side of said IPFi;
b) transforming said parent plasmid into a cell suitable for site-specific recombination to occur, under conditions such that topoisomerase IV decatenation activity is inhibited, thereby producing a plurality of catenated DNA circles, wherein at least one of the circles in each catenane is a supercoiled DNA MiniVector of less than about 5 kb in length;
c) decatenating the catenated site-specific recombination products with a topoisomerase, thereby releasing the supercoiled DNA MiniVector from the catenanes; and
d) isolating the supercoiled DNA MiniVector using PEG precipitation and sequential size exclusion gel-filtration chromatography using resins of differing size range separation.

15. The composition of claim 1, 9 or 14 for use in treating IPF.
